# EUROPEAN PATENT APPLICATION

(11) **EP 1 577 299 A1**
(43) Date of publication of application: **21.09.2005**
(21) Application number: 03814529.8
(22) Date of filing: 01.08.2003
(51) Int. Cl.: C07D 211/96, A61K 31/445, A61P 1/04, A61P 3/10, A61P 11/06, A61P 17/00, A61P 19/02, A61P 29/00, A61P 31/04, A61P 31/18, A61P 37/06, A61P 37/08, A61P 43/00

(54) **ALKYNYL-SUBSTITUTED AZASUGAR DERIVATIVE AND DRUG CONTAINING THE SAME AS THE ACTIVE INGREDIENT**

(30) Priority: 26.12.2002 JP 2002375800
(71) Applicant: Carna Biosciences Inc., Hyogo 650-0047 (JP)
(72) Inventor: TSUKIDA, Takahiro, Osaka-shi, Osaka 534-0016 (JP); MORIYAMA, Hideki, RESIDENCE EMANOLE 17 605, Sapporo-shi, Hokkaido 001-0017 (JP); NISHIMURA, Shinichiro, Sapporo-shi, Hokkaido 060-0009 (JP); INOUE, Yoshimasa, Osaka-shi, Osaka 534-0016 (JP)
(74) Representative: TBK-Patent
(86) International application number: PCT/JP2003/009845
(87) International publication number: WO 2004/060875

(57) **Abstract**

An alkynyl-substituted azasugar derivative and a drug containing the same as an active ingredient are disclosed. This drug is useful as a preventive or remedy for insulin-independent diabetes, rheumatoid arthritis, osteoarthritis, sepsis, acquired immune deficiency syndrome (AIDS), graft-versus-host disease (GVHD), asthma, atopic dermatitis, and ulcerative colitis.

## Description

### TECHNICAL FIELD

The present invention relates to a hydroxamic acid derivative having an azasugar skeleton with acetylene-substituted arylsulfonylamide attached, which functions as a selective inhibitor against a TNF-α converting enzyme (TACE).

### BACKGROUND ART

TNF-α is a kind of cytokine produced by an active macrophage and serves as an inflammatory mediator at the inflammatory site. TNF-α is originally important cytokine. However, when it is excessively produced, it may cause diseases such as insulin-independent diabetes, rheumatoid arthritis, osteoarthritis, sepsis, acquired immune deficiency syndrome (AIDS), graft-versus-host disease (GVHD), asthma, atopic dermatitis, and ulcerative colitis. Therefore, there is a fair chance that a drug capable of inhibiting production of TNF-α (TNF-α production inhibitor) serves as a preventive or remedy for these diseases.

TNF-α is produced as a result of processing of a membrane-anchored precursor having a molecular weight of 26 kDa comprising 233 amino acids with the converting enzyme (TACE). Therefore, intense interest has been shown towards a compound having TACE inhibitory activity (TACE inhibitor) for the purpose of developing a TNF-α production inhibitor.

It has recently become apparent that a group of functional proteins known generically as ADAM (A Disintegrin And Metalloprotease) are involved in processing of various membrane- anchored proteins and also it has been reported that ADAM 17 functions as TACE (see Non-Patent Document 1). It is known that most MMP inhibitors, which have hitherto been synthesized, also function as a TACE inhibitor because an extracellular matrix degrading enzyme, which has hitherto been known as a matrix metalloprotease (MMP), in a connective tissue is a related enzyme of ADAM molecules (see, for example, Patent Document 1).

However, it is pointed out that a MMP1 inhibitor causes arthralgia when administered to human (see Non-Patent Document 2). Therefore, a compound capable of selectively exerting an inhibitory effect on TACE is required as a TNF-α production inhibitor.

PCT International Publication (see Patent Document 2) discloses a hydroxamic acid derivative having an acetylene-substituted arylsulfonylamide site as a TACE inhibitor. Various hydroxamic acid derivatives having a cyclic skeleton such as piperazine are disclosed. However, it is hardly to say that these derivatives have sufficient selectivity against TACE.

### (Patent Document 1)

U.S. Patent No. 5,691,382

### (Patent Document 2)

PCT International Publication WO00/40729

### (Non-Patent Document 1)

Black et al., Nature, 385, 729-733, (1997); Moss, Nature, 385, 733-736 (1997)

### (Non-Patent Document 2)

Scrip, No.2349, p.20 (1998)

### DISCLOSURE OF THE INVENTION

An object of the present invention is to find a compound capable of exerting a selective inhibitory effect on TACE and to provide an excellent TNF-α production inhibitor.

The present inventors have intensively studied and found that a compound represented by the above formula (I) has a selective inhibitory effect on TACE, and thus the present invention has been completed.

The present invention will now be described in detail.

### BEST MODE FOR CARRYING OUT THE INVENTION

The present invention is directed to a compound represented by the general formula (I): 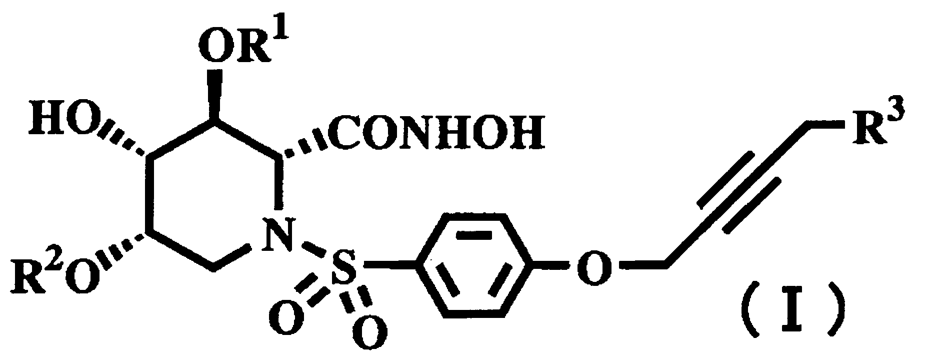 wherein R¹ and R² represent a hydrogen atom, a C₁-C₈ alkyl group, a C₃-C₈ alkenyl group or a benzyl group and at least one of R¹ and R² is a hydrogen atom, and R³ represents a hydrogen atom or a hydroxyl group, or a pharmaceutically acceptable salt thereof, and a drug containing the same as an active ingredient.

The C₁-C₈ alkyl group means a linear or branched alkyl group having 1 to 8 carbon atoms and is particularly preferably a lower alkyl group having 1 to 4 carbon atoms, such as methyl group or ethyl group.

The C₃-C₈ alkenyl group means a linear or branched alkenyl group having 3 to 8 carbon atoms and is particularly preferably a lower alkenyl group having 3 to 4 carbon atoms, such as allyl group or 2-methylallyl group.

Although plural asymmetric carbons exist in the compound (I), the asymmetric carbon constituting the piperidine ring is limited to those represented by stereochemistry shown above. In case the asymmetric carbon exists in an alkyl group selected as R¹ and R², all stereoisomers based on the asymmetric carbon or a mixture thereof are included.

Compounds represented by the following formula (IIA) or (IIB) : 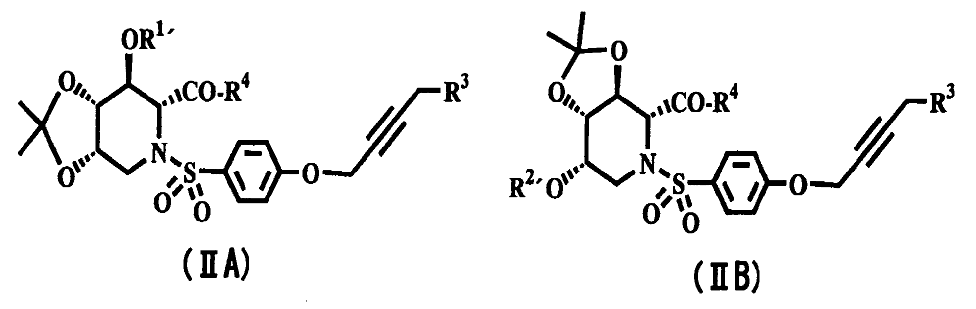 wherein R¹' and R²' represent a hydrogen atom, a C₁-C₈ alkyl group, a C₃-C₈ alkenyl group or a benzyl group, R³ represents a hydrogen atom or a hydroxyl group, and CO-R⁴ represents a hydroxamic acid equivalent, are also parts of the present invention.

As used herein, the hydroxamic acid equivalent means a monovalent atomic group -CONHOH in itself, and a functional group which can be chemically converted into the atomic group -CONHOH by a conventional method. R⁴ includes, for example, methoxy group, ethoxy group or benzyloxyamino group. Furthermore, since a benzyloxyamino group can be introduced easily by the condensation reaction of carboxylic acid and benzyloxyamine, the hydroxamic acid equivalent includes various ester groups which can be converted easily into a carboxyl group, in addition to the carboxyl group. Therefore, various carboxylic acids protected with a carboxyl protecting group are included, and specific examples of R⁴ include benzyloxy group and tert-butyloxy group, in addition to the above-mentioned methoxy group, ethoxy group and benzyloxyamino group.

Abbreviations and symbols used herein are as follows.
Ag₂O: silver oxide
Bn: benzyl group
CeCl₃: cerium chloride heptahydrate
DMF: N,N-dimethylformamide
DMP: 2,2-dimethoxypropane
DMAP: 4-dimethylaminopyridine
DIEA: diisopropylethylamine
K₂CO₃: potassium carbonate
THF: tetrahydrofuran
Pd/C: palladium-carbon
Pd(OH)₂/C: palladium hydroxide-carbon
PPh₃: triphenylphosphine
NaCN: sodium cyanide
NH₂OH: aqueous 50% hydroxylamine solution
TsOH: p-toluenesulfonic acid monohydrate
MsCl: mesyl chloride
TBAF: 1M tetrabutylammonium fluoride/THF solution
TBDMS: tert-butyldimethylsilyl group

The method of preparing a compound (I) of the present invention will now be described.

### (1) Synthesis of common intermediate (IIB-3)

A compound (IIB-3) can be prepared, for example, through the following scheme (Scheme 1): 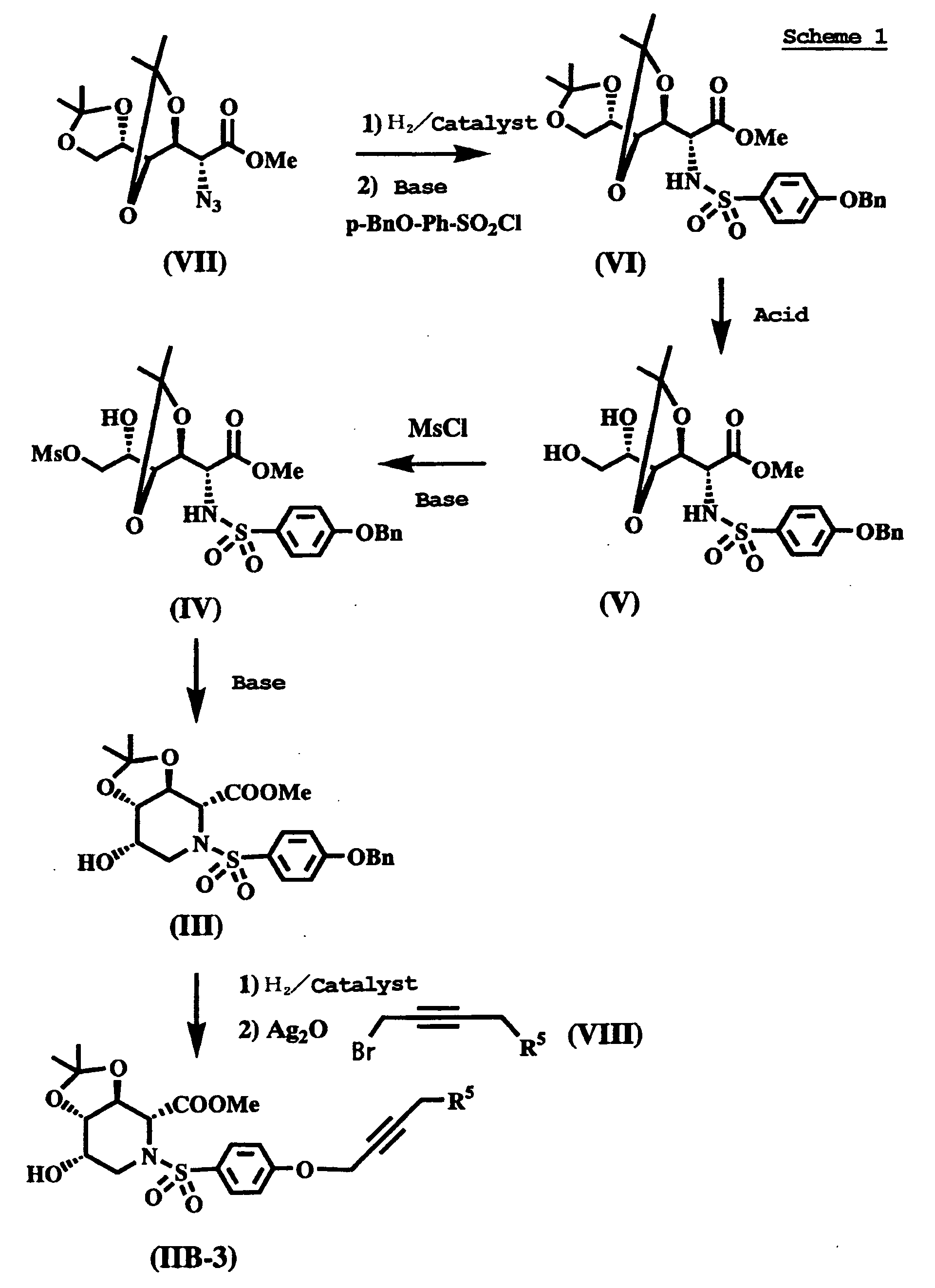 wherein R⁵ represents a hydrogen atom or a tert-butyldimethylsilanyloxy group.

First, an azide group of a compound (VII) [see Synthesis, No. 9, pp 1305-1309 (2000)] is reduced and then the compound is reacted with p-benzyloxybenzenesulfonyl chloride to obtain a compound (VI). For example, the azide group is reduced in ethyl acetate, lower alcohol or 1,4-dioxane under hydrogen gas flow or pressure at a temperature of room temperature to 60°C in the presence of a catalyst such as 10% palladium-carbon, 20% palladium hydroxide-carbon or platinum. The subsequent reaction with p-benzyloxybenzenesulfonyl chloride is usually conducted by stirring in an inert solvent such as DMF at a temperature of 0°C to room temperature for 1 to 24 hours in the presence of a base such as DMAP.

Then, a terminal acetonide group of the compound (VI) is selectively cleaved and removed. The reaction can be conducted, for example, by stirring in a solvent such as methanol at room temperature to 50°C for 5 hours to 4 days in the presence of a cation exchange resin, optionally adding water. In this case, the reaction can be also conducted by the following method, in addition to the above-mentioned method using the cation exchange resin. For example, there can be exemplified a method of stirring in acetonitrile at room temperature for 0.5 to 2 hours, using cerium chloride heptahydrate and oxalic acid, to obtain the objective product (V).

Then, only a primary hydroxyl group of the compound (V) is selectively converted into a mesyl group. The reaction is conducted, for example, by stirring in a solvent such as methylene chloride preferably at a low temperature of -60 to -20°C for 30 minutes to 5 hours in the presence of a base such as triethylamine or DIEA, using 0.95 to 1.05 mols of mesyl chloride, to obtain the objective compound (IV).

Then, a compound (III) is obtained by the intramolecular ring closure reaction of the compound (IV). The reaction is usually conducted in an inert solvent such as DMF at room temperature to 100°C, preferably 40 to 60°C, for 1 to 5 hours in the presence of a base such as potassium carbonate or triethylamine to obtain the objective compound (III).

Finally, the compound (III) is debenzylated by hydrogenolysis and reacted with a bromide (VIII) to obtain the objective common intermediate (IIB-3). This hydrogenolysis is usually conducted by stirring in ethyl acetate, a lower alcohol such as methanol, or 1,4-dioxane under hydrogen gas flow or pressure at a temperature of room temperature to 60°C for 1 to 5 hours in the presence of a catalyst such as 10% palladium-carbon, 20% palladium hydroxide-carbon or platinum, optionally adding water. The subsequent reaction with the compound (VIII) is usually conducted in a solvent such as methylene chloride at a temperature of room temperature to 50°C for 5 to 24 hours in the presence of silver oxide to obtain the objective compound (IIB-3).

### (2) Preparation of 4,5-dihydroxy compound (Ia)

A compound (Ia) can be prepared, for example, through the following scheme (Scheme 2): 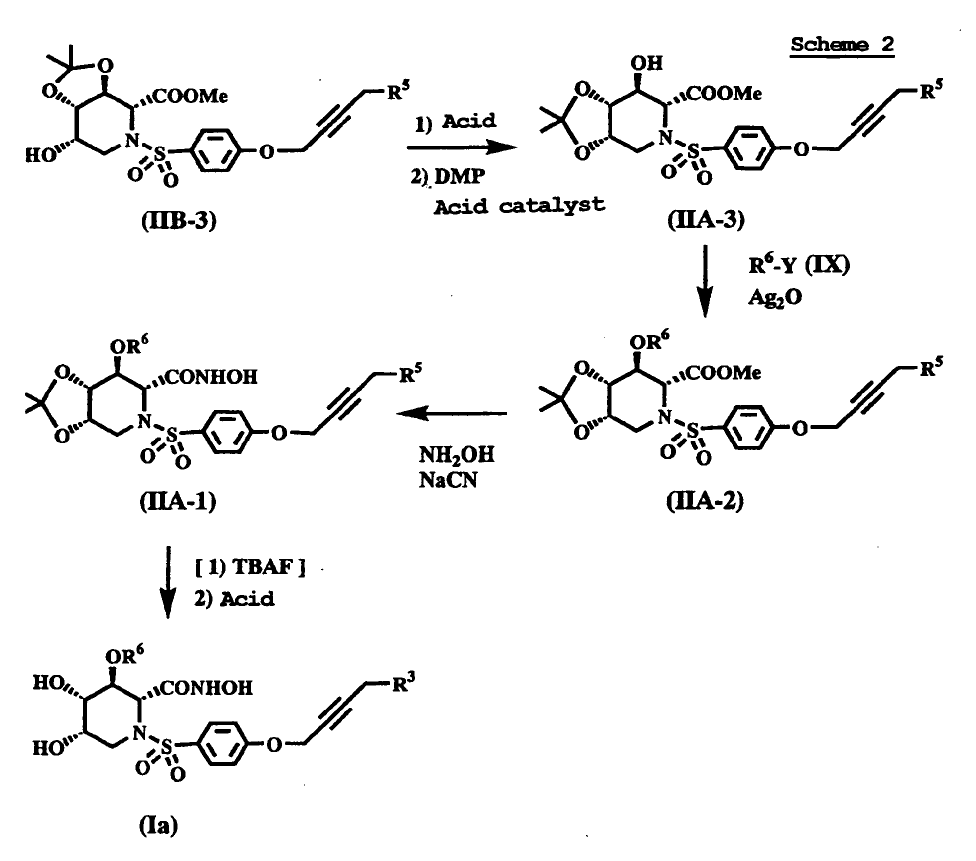 wherein R⁶ represents a C₁-C₈ alkyl group, a C₃-C₈ alkenyl group or a benzyl group, Y represents a bromine atom or an iodine atom, and R³ and R⁵ are as defined above.

First, an acetonide group of the common intermediate (IIB-3) shown in Scheme 1 is cleaved and removed, and then recombination of the acetonide group is conducted to obtain a compound (IIA-3). The cleavage of the acetonide group can be conducted by the above-mentioned method which uses a cation exchange resin. The reprotection of the acetonide group can be conduced, for example, by adding DMP in an inert solvent such as DMF in the presence of an acid catalyst such as p-toluenesulfonic acid monohydrate or camphor-10-sulfonic acid, and reacting at a temperature of room temperature to 80°C for 2 to 24 hours.

Then, the compound (IIA-3) is reacted with a compound (IX) to obtain a compound (IIA-2). The reaction is usually conducted in a solvent such as methylene chloride at a temperature of room temperature to 50°C for 24 hours to 7 days in the presence of silver oxide to obtain the objective compound (IIA-2).

Then, a compound (IIA-1) is obtained by aminolysis of the compound (IIA-2) using hydroxylamine. The reaction is usually conducted by stirring in a lower alcohol such as methanol at room temperature for 24 hours to 3 days in the presence of NaCN.

Finally, the acetonide group of the compound (IIA-1) is cleaved and removed to obtain the objective compound (Ia). The reaction can be conducted by the above-mentioned method which uses a cation exchange resin. At this time, when R⁵ is a tert-butyldimethylsilanyloxy group, the acetonide group is cleavaged and removed after deprotecting the tert-butyldimethylsilyl group. The deprotection of the tert-butyldimethylsilyl group can be usually conducted by optionally adding an acid such as acetic acid in an inert solvent such as THF in the presence of TBAF and reacting at a temperature of room temperature to 50°C for 2 to 24 hours.

When R⁶ is a propyl group or a related group thereof, there can be used another method shown in the following Scheme 3: 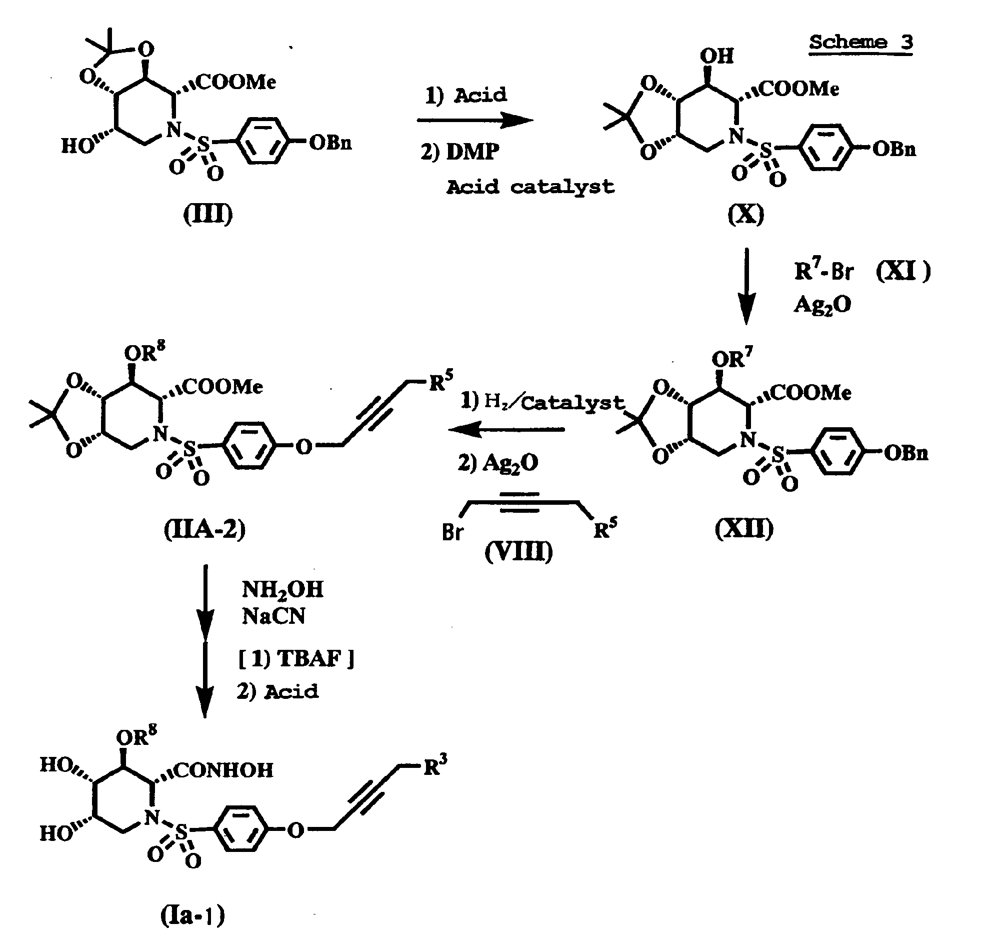 wherein R⁷ represents an allyl group or a substituted allyl group, and R⁸ represents a propyl group or a reduced substituted allyl group.

That is, it is a method of replotecting the acetonide group of the compound (III), reacting with allyl bromide or substituted allylbromide (XI) and introducing a butynyl group. In the process of cleavage and removal of a benzyl group in a compound (XII), a carbon-carbon double bond of the allyl group is reduced, and thus there can be prepared the objective product (Ia-1) of the formula (Ia) in which R⁶ is a propyl group or a related group thereof.

### (3) Preparation of 3,4-dihydroxy compound (Ib)

A compound (Ib) can be prepared, for example, through the following scheme (Scheme 4): 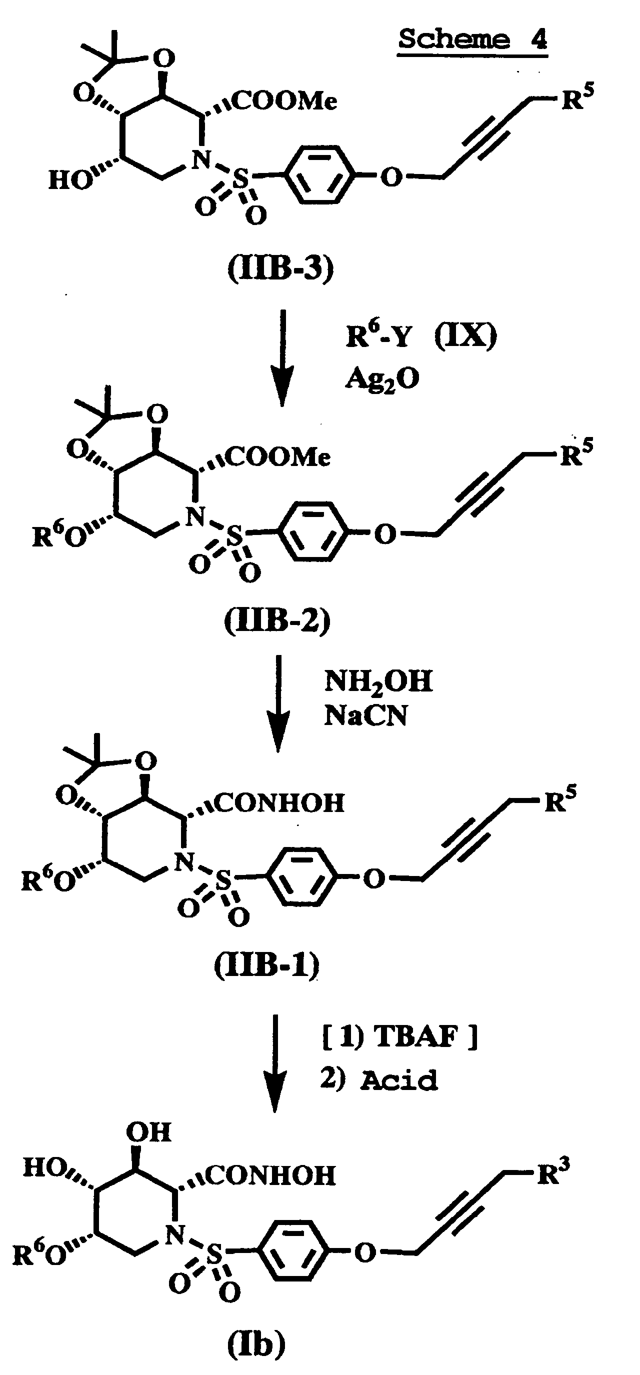 wherein R³, R⁵, R⁶ and Y are as defined above.

First, the common intermediate (IIB-3) shown in Scheme 1 is reacted with a compound (IX) to obtain a compound (IIB-2). The reaction is usually conducted in a solvent such as methylene chloride at a temperature of room temperature to 50°C for 24 hours to 7 days in the presence of silver oxide to obtain the objective compound (IIB-2).

Then, a compound (IIB-1) is obtained by aminolysis of the compound (IIB-2) using hydroxylamine. The reaction can be conducted by the above-mentioned method.

Finally, the compound (IIB-1) is deprotected to obtain the objective compound (Ib). The deprotection can be conducted in the same manner as shown in Scheme 2.

When R⁶ is a propyl group or a related group thereof in the compound (Ib), another method as shown in Scheme 3 can be used, similar to the case of the compound (Ia). That is, it is a method of reacting the compound (III) with allyl bromide or substituted allylbromide (XI) and introducing a butynyl group. In the process of cleavage and removal of a benzyl group, a carbon-carbon double bond of the allyl group is reduced, and thus there can be prepared the objective product of the formula (Ib) in which R⁶ is a propyl group or a related group thereof.

### (4) Preparation of 3,4,5-trihydroxy compound (Ic)

A compound (Ic) can be prepared, for example, through the following scheme (Scheme 5): 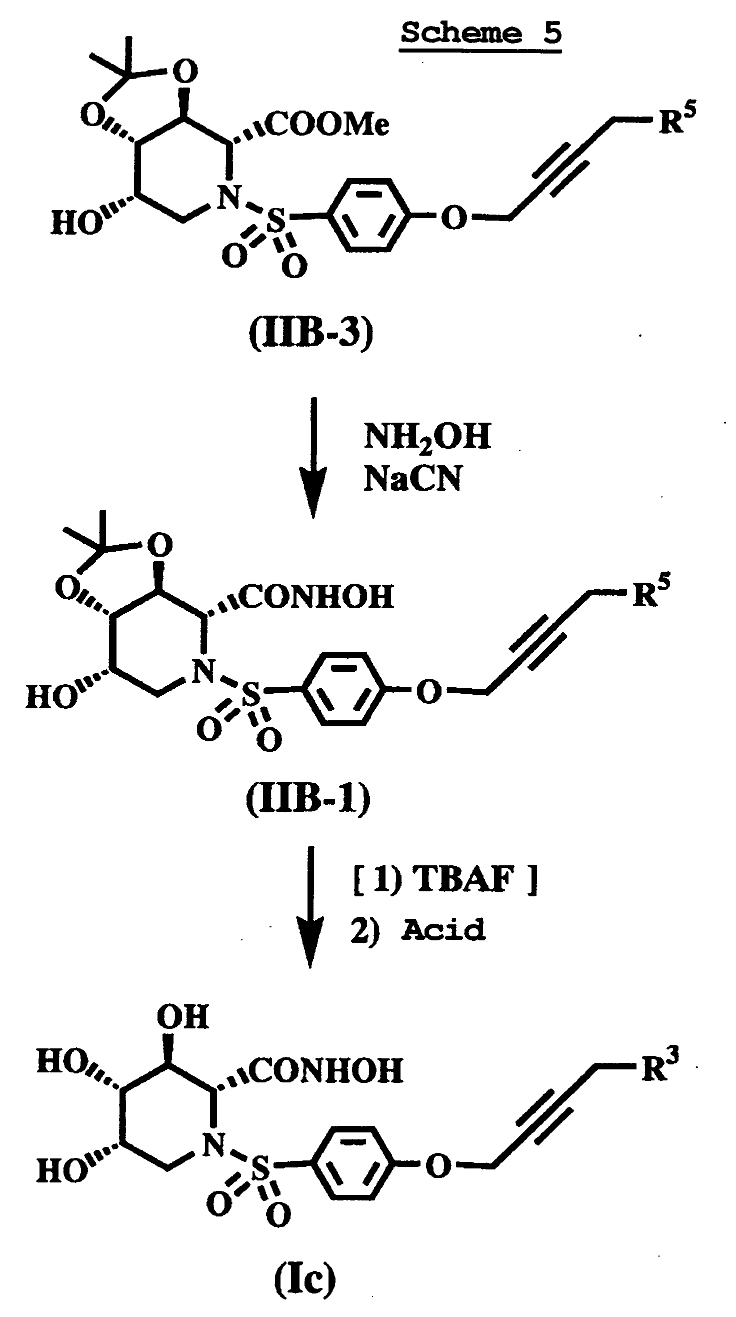 wherein R³ and R⁵ are as defined above.

First, a compound (IIB-4) is obtained by aminolysis of the common intermediate (IIB-3) shown in Scheme 1 and hydroxylamine. The reaction is usually conducted by stirring in a lower alcohol such as methanol at room temperature for 24 hours to 4 days in the presence of NaCN.

Finally, the compound (IIB-4) is deprotected to obtain the objective compound (Ic). The reaction can be conducted by the above-mentioned method.

These compounds can be administered orally or parenterally to human.

Examples of oral dosage form include solid preparations such as tablets, granules, powders, fine granules and hard capsules; and liquid preparations such as syrups and soft capsules. These preparations can be prepared by a conventional method. Tablets, granules, powders or fine granules are prepared by mixing the above-mentioned compound or pharmaceutically acceptable salt thereof with commonly used pharmaceutical additives such as lactose, starch, crystalline cellulose, magnesium stearate, hydroxypropylcellulose and talc, while hard capsules are prepared by filling these fine granules or powders into a capsule. Syrups are prepared by dissolving or suspending the above-mentioned compound or pharmaceutically acceptable salt thereof in an aqueous solution containing sucrose or carboxycellulose, while soft capsules are prepared by dissolving or suspending the above-mentioned compound or pharmaceutically acceptable salt thereof in a lipid excipient such as vegetable oil, oily emulsion or glycol, and filling the resulting solution or suspension in a soft capsule.

Examples of parenteral dosage form include injections; external preparations such as ointments, lotions and creams; suppository preparations such as suppositories and pessaries; and pernasal preparations such as aerosols. These preparations can be prepared by a conventional method and, for example, injections are prepared by dissolving or emulsifying the above-mentioned compound or pharmaceutically acceptable salt thereof in physiological saline or a lipid excipient such as vegetable oil, oily emulsion or glycol, and sterilely filling the resulting solution or emulsion in an ampoule or a vial. Ointments are prepared, for example, by a conventional method of adding the above-mentioned compound or pharmaceutically acceptable salt thereof to a base such as vaseline, paraffin or glycerin, and optionally adding emulsifiers or preservatives.

The dose of the drug of the present invention varies depending on the dosage form, age, sex or weight of patients, or conditions of diseases, but a dairy dose of the active ingredient is usually within a range from 0.1 to 600 mg/kg, and preferably from 10 to 200 mg/kg. The drug is administered once or 2 to 4 times per day.

### Test Example 1

Measurement of matrix metalloprotease (MMP) inhibitory activity

### 1. Test compound

Compound (a) of the present invention: (2R,3S,4S,5S)-1-(4'-but-2'-ynyloxybenzenesulfonyl)-4,5-dihydroxy-3-methoxypiperidine-2-carboxylic acid hydroxamide (Compound of Example 2)
Compound (b) of the present invention: (2R,3S,4S,5S)-3-benzyloxy-1-(4'-but-2'-ynyloxybenzenesulfonyl)-4,5-dihydroxypiperidine-2-carboxylic acid hydroxamide (Compound of Example 3)
Compound (c) of the present invention: (2R, 3S, 4R, 5S)-1-(4'-but-2'-ynyloxybenzenesulfonyl)-3,4-dihydroxy-5-methoxypiperidine-2-carboxylic acid hydroxamide (Compound of Example 4)
Compound (d) of the present invention: (2R,3S,4S,5S)-1-(4'-but-2'-ynyloxybenzenesulfonyl)-3,4,5-trihydroxy-piperidine-2-carboxylic acid hydroxamide (Compound of Example 5)
Compound (e) of the present invention: (2R,3S,4S,5S)-1-(4'-but-2'-ynyloxybenzenesulfonyl)-4,5-dihydroxy-3-ethoxypiperidine-2-carboxylic acid hydroxamide (Compound of Example 9)
Compound (f) of the present invention: (2R,3S,4S,5S)-4,5-dihydroxy-1-[4'-(4"-hydroxy-but-2'-ynyloxy)benzenesulfonyl]-3-methoxy-piperidine-2-carboxylic acid hydroxamide (Compound of Example 10)

### 2. Test Procedure

A DNA fragment coding for catalytic regions in MMP-1 and MMP-9 was amplified by a PCR method with cDNA prepared from HT-1080 cells derived from human fibrosarcoma. In the same manner, a DNA fragment containing a pro-region and a catalyst region in MMP-3 was also prepared. The amplified DNA fragment was cloned into a commercially available expression vector which was designed to allow a histidine tag to add to the N-terminal, and then transformed into E. coli. The resulting transformants were cultured and a recombinant MMP was purified from a cell lysate using Ni-NTA regin (QIAGEN INC.). Recombinant MMP-3 was further activated by reacting with 1 mM para-aminophenyl mercury acetate at 37°C for one hour.

The inhibitory activity was measured according to the method by Night et al. [FEBS Lett. 296, p263 (1992)] or the method by Nagase et al. [J. Biol. Chem. 269, p20952 (1994)] using a 96-half well black microplate. A solution prepared by dissolving each of test compounds (a) to (f) in dimethyl sulfoxide was diluted with a reaction buffer [50 mM Tris-HCl (pH 7.5) buffer containing 150 mM NaCl, 10 mM CaCl₂, 0.05% Brij-35®] and then 25 µL of the dilute solution was added in a well.

After 25 µL of a MMP solution diluted with the reaction buffer was added thereto, the mixture was incubated at 37°C for 10 minutes. Then, the reaction was initiated by adding 50 µL of a 10 µM fluorescence quenching peptide substrate prepared from the reaction buffer. As the fluorescence quenching peptide substrate, MCA-Pro-Leu-Gly-Leu-DPA-Ala-Arg-NH₂ (MCA = 7-methoxycoumarin-4-acetyl and DPA = N-3-(2,4-dinitrophenyl)-L-2,3-diaminopropionyl) were used for MMP-1 and MMP-9, and MCA-Arg-Pro-Lys-Pro-Val-Nva-Trp-Arg-Lys (DNP)-NH₂ (DNP = 2,4-dinitrophenyl) was used for MMP-3. A fluorescence intensity (excitation 320/fluoresecence 405 nm) immediately after the initiation of the reaction and a fluorescence intensity after the reaction at 37°C (for 2 hours for MMP-1 and MMP-3, for 3 hours for MMP-9) were measured and a difference in fluorescence intensity was taken as an indicator of enzymatic activity. The inhibition rate (%) at each concentration of an inhibitor was calculated and an IC50 value was determined from the dose-response curve. From this IC50 value and previously determined Km value for a TACE substrate, a Ki value was calculated using GraphPad Prism software (GraphPad Software, Inc.).

### 3. Results

The results are shown in Table 1, along with the results of Test Example 2.

### Test Example 2

Measurement of inhibitory activity against TNF-α converting enzyme (TACE)

### 1. Test compounds

the same as in case of Test Example 1

### 2. Test procedure

A full length cDNA for TACE was cloned from THP-1 cells derived from human acute monocytic leukemia and a DNA fragment cording a signal region, a pro-region and a catalytic region was amplified by a PCR method. An appropriate restriction enzyme recognizing sequence was added to both terminals of the amplified fragment and also a sequence of an antigenic octapeptide (FLAG™) tag was inserted in the 3' terminal. A recombinant bacmid was prepared from the resulting amplified fragment using a pFastBac system (Life Technologies) and then transfected into insect cells to generate a recombinant baculovirus. Insect cells were infected with the recombinant baculovirus and the recombinant TACE released into culture supernatant was purified by an affinity gel column.

The inhibitory activity was measured according to the method by Van Dyke et al. [Bioorg. Med. Chem. Lett. 7, p1219 (1997)] using a 96 half well black microplate. A solution prepared by dissolving each of test compounds (a) to (f) in dimethylsulfoxide was diluted with a reaction buffer [20 mM tris-HCl buffer (pH 7.5) containing 0.05% Brij-35®] and then 40 µL of the dilute solution was added in a well. After 40 µL of a TACE solution (125 ng/mL) diluted with the reaction buffer was added thereto, the mixture was incubated at 37°C for 10 minutes. Then, the reaction was initiated by adding 20 µL of a 25 µM fluorescence quenching peptide substrate MCA-Pro-Leu-Ala-Glu-Ala-Val-DPA-Arg-Ser-Ser-Ser-Arg-NH₂ prepared from the reaction buffer. A fluorescence intensity (excitation 320 nm/fluoresecence 405 nm) immediately after the initiation of reaction and a fluorescence intensity after the reaction at 37°C for 30 minutes were measured and a difference in fluorescence intensity was taken as an indicator of enzymatic activity. The inhibition rate (%) at each concentration of an inhibitor was calculated and an IC50 value was determined from the dose-response curve. From this IC50 value and previously determined Km value for a TACE substrate, a Ki value was calculated using data processing software (GraphPad Prism™, GraphPad Software, Inc.).

### 3. Test results

The test results are shown in Table 1.

**(Table 1)**

| Test compounds | Ki (nM) | | | |
|---|---|---|---|---|
| | MMP1 | MMP3 | MMP9 | TACE |
| Compound (a) of the present invention | > 850 | > 650 | > 790 | 4.3 |
| Compound (b) of the present invention | > 850 | > 650 | > 790 | 9.4 |
| Compound (c) of the present invention | > 850 | > 650 | > 790 | 2.4 |
| Compound (d) of the present invention | > 850 | 490 | > 790 | 3.4 |
| Compound (e) of the present invention | > 850 | > 650 | > 790 | 8.5 |
| Compound (f) of the present invention | > 850 | > 650 | > 790 | 18 |

Any compound of the present invention selectively exerts an inhibitory effect on TACE.

### EXAMPLES

The present invention will now be described in detail by way of Reference Examples and Examples.

### Reference Example 1

### Preparation of 4-(bromo-but-2-ynyloxy)-tert-butyldimethylsilane:

### (1) 4-(tert-butyl-dimethylsilanyloxy)-but-2-yne-1-ol:

But-2-yne-1,4-diol (5 g) was dissolved in DMF (60 mL) and imidazole (11.07 g) was added, followed by the addition of tert-butyldimethylsilyl chloride (8.75 g) with stirring under ice cooling and further stirring at room temperature overnight. Ether (500 mL) was added and the reaction solution was washed with water, and then the organic layer was dried over magnesium sulfate and the solvent was distilled off under reduced pressure. The resulting residue was purified by silica gel medium pressure column chromatography (ethyl acetate:cyclohexane = 1:4 → 1:3) to obtain the titled compound (4.38 g) as a syrup.
¹H-NMR (CDCl₃) δ: 0.12 (s, 6H), 0.92 (s, 9H), 1.55-1.7 (m, 1H), 4.2-4.3 (m, 2H), 4.35 (d, 1H, J = 1.0 Hz).

### (2) 4-(bromo-but-2-ynyloxy)-tert-butyldimethylsilane:

The compound (1.5 g) of (1) was dissolved in methylene chloride (40 mL) and PPh₃ (2.95 g) was added, followed by the addition of carbon tetrabromide (3.73 g) and further stirring at room temperature for 30 minutes. The solvent was distilled off under reduced pressure and the resulting residue was purified by silica gel medium pressure column chromatography (ethyl acetate:cyclohexane = 1:4) to obtain the titled compound (2.45 g) as a syrup.
¹H-NMR (CDCl₃) δ: 0.13 (s, 6H), 0.91 (s, 9H), 3.96 (s, 2H), 4.37 (s, 2H).

### Example 1

### Preparation of (3aS,4R,7S,7aS)-5-(4'-but-2'-ynyloxybenzenesulfonyl)-7-hydroxy-2,2-dimethyl-hexahydro-[1,3]dioxolo[4,5-c]pyridine-4-carboxylic acid methyl ester:

### (1) (2R,4'R,4"S,5'S)-(benzyloxybenzenesulfonylamino)-(2',2',2",2"-tetramethyl-[4',4"]bis[[1,3]dioxolanyl]-5'-yl)-acetic acid methyl ester:

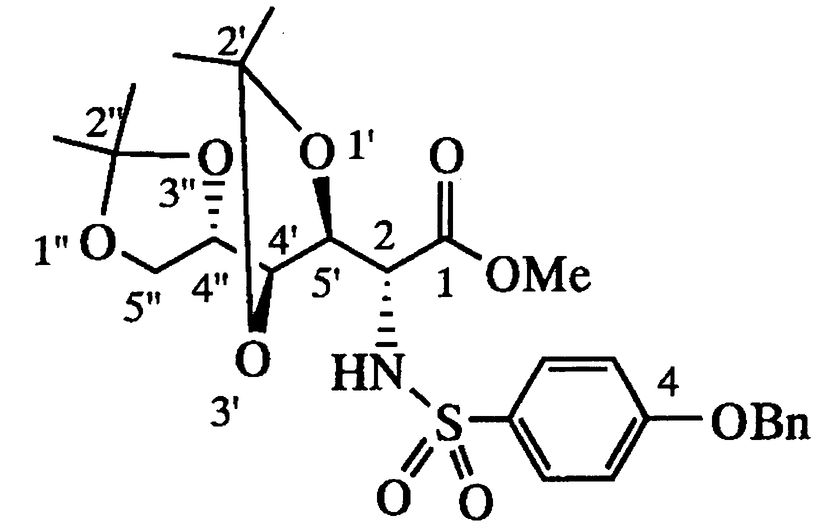

A known compound [(2R,4'R,4"S,5'S)-azide-(2',2',2",2"-tetramethyl-[4',4"]bis[[1,3]dioxolanyl]-5'-yl)-acetic acid methyl ester, 49.5 g] was dissolved in ethyl acetate (300 mL) and 10% Pd/C (4.7 g) was added, followed by stirring under hydrogen pressure at 40°C for 4 hours. The catalyst was removed by filtration and the filtrate was concentrated under reduced pressure. The resulting residue was dissolved in DMF (450 mL) and DMAP (19.2 g) and p-benzyloxybenzenesulfonyl chloride (44.4 g) were added, followed by stirring at room temperature overnight. The reaction solution was mixed with ethyl acetate (700 mL) and then washed in turn with IN hydrochloric acid, water and saturated saline. The organic layer was dried over magnesium sulfate and the solvent was distilled off under reduced pressure. The resulting residue was purified by silica gel medium pressure column chromatography (ethyl acetate:cyclohexane = 1:2) to obtain the titled compound (65 g) as a colorless crystal.
¹H-NMR (CDCl₃)δ: 1.29 (s, 3H), 1.33 (s, 3H), 1.37 (s, 3H), 1.47 (s, 3H), 3.52 (s, 3H), 3.9-4.05 (m, 3H), 4.1-4.2 (m, 3H), 5.15 (s, 2H), 5.57 (d, 1H, J = 6.7 Hz), 7.04 (d, 2H, J = 8.9 Hz), 7.35-7.45 (m, 5H), 7.79 (d, 2H, J = 8.9 Hz).

### (2) (1"S,2R,4'S,5'R)-(4-benzyloxybenzenesulfonylamino)-[5'-(1",2"-dihydroxy-ethyl)-2',2'-dimethyl-[1,3]dioxolan-4'-yl]-acetic acid methyl ester:

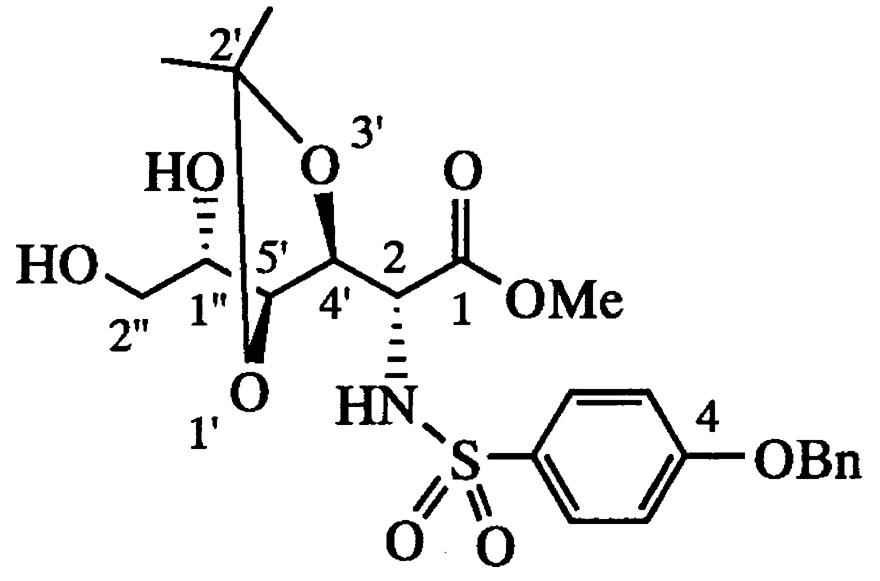

The compound (65 g) of (1) was dissolved in acetonitrile (500 mL) and CeCl₃ (90.4 g) and oxalic acid (545 mg) were added, followed by stirring at room temperature for one hour. After adding sodium carbonate until the reaction solution is neutralized, the insoluble material was removed by filtration and the residue was washed with ethyl acetate. The filtrate and washing were combined and the solvent was distilled off under reduced pressure. The resulting residue was purified by silica gel medium pressure column chromatography (ethyl acetate:cyclohexane = 1:1 → 3:2, followed by chloroform:methanol = 10:1) to obtain the titled compound (29.9 g), and thus a starting material (18.5 g) was recovered.
¹H-NMR (CDCl₃) δ: 1.32 (s, 3H), 1.36 (s, 3H), 2.55 (bs, 1H), 3.32 (bs, 1H), 3.50 (s, 3H), 3.7-3.8 (m, 2H), 3.85-3.9 (m, 1H), 4.00 (t, 1H, J = 7.0 Hz), 4.1-4.2 (m, 1H), 4.25-4.3(m, 1H), 5.14(s, 2H), 6.12 (d, 1H, J = 7.7 Hz), 7.05 (d, 2H, J = 8.9 Hz), 7.3-7.5 (m, 5H), 7.78 (d, 2H, J = 8.9 Hz).

### (3) (1"R,2R,4'S,5'R)-(4-benzyloxybenzenesulfonylamino)-[5'-(1"-hydroxy-2"-methanesulfonyloxy-ethyl)-2',2'-dimethyl-[1,3]dioxolan-4'-yl]-acetic acid methyl ester:

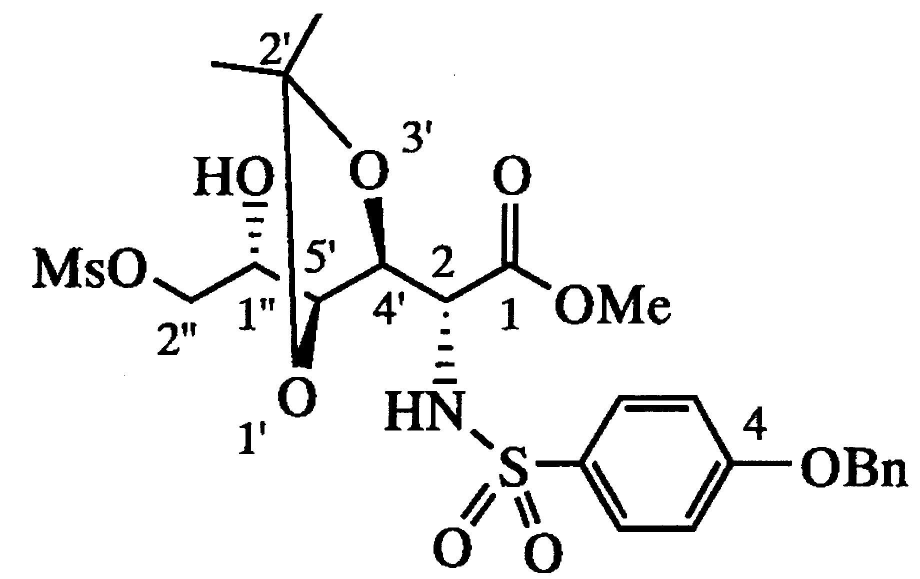

The compound (38.9 g) of (2) was dissolved in methylene chloride (320 mL) and triethylamine (8.7 g) was added. The mixture was cooled to -40°C and mesyl chloride (8.96 g)/methylene chloride (10 mL) were rapidly added dropwise, followed by stirring at the same temperature for 30 minutes. The reaction solution was washed with saturated saline and the organic layer was dried over magnesium sulfate, and then the solvent was distilled off under reduced pressure. The resulting residue was purified by silica gel medium pressure column chromatography (ethyl acetate:cyclohexane = 1:2 → 1:1 → 2:1 → 4:1) to obtain the titled compound (19.8 g), and thus a raw material (9.9 g) was recovered.
¹H-NMR (CDCl₃)δ: 1.31 (s, 3H), 1.36 (s, 3H), 3.12 (s, 3H), 3.54 (s, 3H), 3.85-3.95 (m, 1H), 3.97 (dd, 1H, J = 6.8, 8.6 Hz), 4.17 (dd, 1H, J = 4.8, 8.4 Hz), 4.26 (dd, 1H, J = 4.9, 6.7 Hz), 4.30 (dd, 1H, J = 6.1, 11.1 Hz), 4.50 (dd, 1H, J = 2.3, 11.1 Hz), 5.15 (s, 2H), 5.73 (d, 1H, J = 8.4 Hz), 7.06 (d, 2H, J = 9.0 Hz), 7.3-7.5 (m, 5H), 7.78 (d, 2H, J = 9.0 Hz).

### (4) (3aS,4R,7S,7aS)-5-(4'-benzyloxybenzenesulfonyl)-7-hydroxy-2,2-dimethyl-hexahydro-[1,3]dioxolo[4,5-c]pyridine-4-carboxylic acid methyl ester:

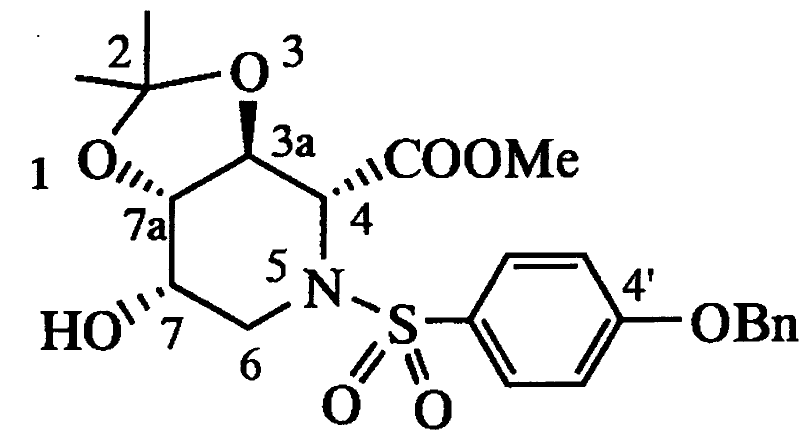

The compound (24.1 g) of (3) was dissolved in DMF (200 mL) and potassium carbonate (5.8 g) was added, followed by stirring at 50°C for 1.5 hours. The reaction solution was mixed with ethyl acetate (700 mL) and then washed with water (× 2) and saturated saline. The organic layer was dried over magnesium sulfate and the solvent was distilled off under reduced pressure. The resulting residue was purified by silica gel medium pressure column chromatography (ethyl acetate:cyclohexane = 1:2 → 2:3) to obtain the titled compound (12.8 g) as a syrup.
¹H-NMR (CDCl₃)δ: 1.44 (s, 3H), 1.46 (s, 3H), 2.27 (bs, 1H), 3.21 (dd, 1H, J = 5.8, 14.7 Hz), 3.48 (dd, 1H, J = 4.5, 9.8 Hz), 3.72 (s, 3H), 3.88 (d, 1H, J = 7.2, 14.7 Hz), 4.05-4.2 (m, 1H), 4.35-4.4 (m, 1H), 4.43 (d, 1H, J = 9.0 Hz), 5.13 (s, 2H), 7.05 (d, 2H, J = 8.9 Hz), 7.3-7.45 (m, 5H), 7.79 (d, 2H, J = 8.9 Hz).

### (5) (3aS,4R,7S,7aS)-5-(4'-but-2'-ynyloxybenzenesulfonyl)-7-hydroxy-2,2-dimethyl-hexahydro-[1,3]dioxolo[4,5-c]pyridine-4-carboxylic acid methyl ester:

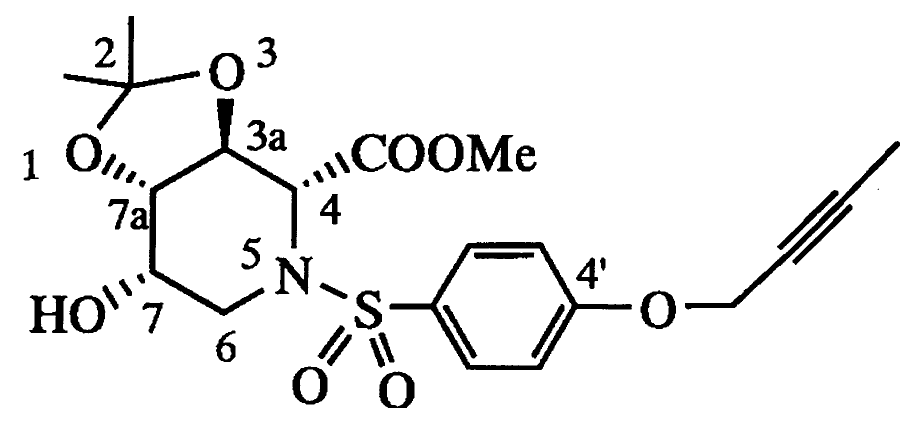

The compound (2.13 g) of (4) was dissolved in ethyl acetate (20 mL) and 10% Pd/C (200 mg) was added, followed by stirring under hydrogen atmosphere at room temperature for 4 hours. The catalyst was removed by filtration and the filtrate was concentrated under reduced pressure. The resulting residue was dissolved in methylene chloride (30 mL) and 1-bromo-2-butyne (655 mg) and silver oxide (1.35 g) were added, followed by stirring under nitrogen atmosphere overnight. The insoluble material was removed by filtration and the filtrate was concentrated under reduced pressure. The resulting residue was purified by silica gel medium pressure column chromatography (ethyl acetate:cyclohexane = 1:2 → 2:3) to obtain the titled compound (2.3 g) as a syrup. ¹H-NMR (CDCl₃)δ: 1.46 (s, 3H), 1.48 (s, 3H), 1.88 (t, 3H, J = 2.3 Hz), 2.24 (s, 1H), 3.23 (dd, 1H, J = 5.8, 14.7 Hz), 3.50 (dd, 1H, J = 4.5, 9.8 Hz), 3.76 (s, 3H), 4.05-4.2 (m, 2H), 4.4-4.45 (m, 1H), 4.46 (d, 1H, J = 9.0 Hz), 4.73 (q, 1H, J = 2.3 Hz), 7.07 (d, 2H, J = 9.0 Hz), 7.82 (d, 2H, J = 9.0 Hz).

### Example 2

### Preparation of (2R,3S,4S,5S)-1-(4'-but-2'-ynyloxybenzenesulfonyl)-4,5-dihydroxy-3-methoxypiperidine-2-carboxylic acid hydroxamide:

### (1) (3aS,6R,7S,7aR)-5-(4'-but-2'-ynyloxybenzenesulfonyl)-7-hydroxy-2,2-dimethyl-hexahydro-[1,3]dioxolo[4,5-c]pyridine-6-carboxylic acid methyl ester:

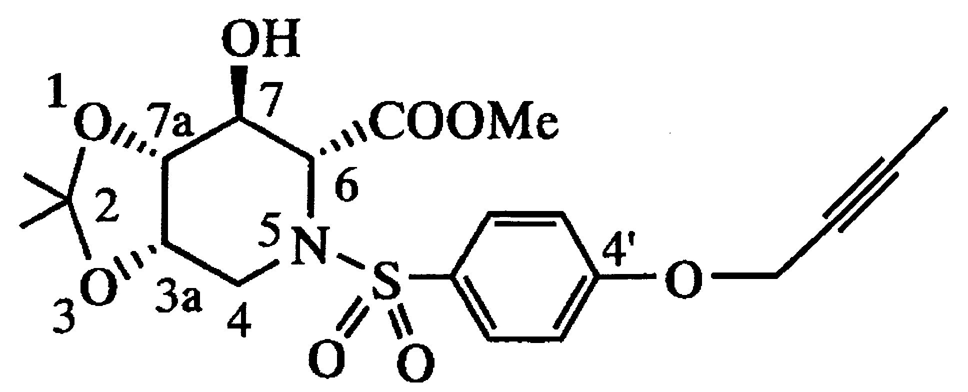

The compound (4.3 g) of Example 1 was dissolved in methanol (40 mL) and a cation exchange resin (MUROMAC, 8.5 g) was added, followed by stirring at room temperature overnight. The insoluble material was removed by filtration and the filtrate was concentrated under reduced pressure. The resulting residue was dissolved in DMF (50 mL) and DMP (10 g) and p-toluenesulfonic acid mononhydrate (150 mg) were added, followed by stirring at room temperature overnight and further stirring at 50°C for 4.5 hours. The reaction solution was distilled off under reduced pressure and the resulting residue was purified by silica gel medium pressure column chromatography (ethyl acetate:n-hexane = 2:3 → 1:1) to obtain the titled compound (3.48 g) as a syrup.
¹H-NMR (CDCl₃)δ: 1.39 (s, 3H), 1.59 (s, 3H), 1.88 (t, 3H, J = 2.3 Hz), 2.50 (d, 1H, J = 6.5 Hz), 3.23 (dd, 1H, J = 9.2, 13.6 Hz), 3.66 (s, 3H), 3.87 (dd, 1H, J = 7.3, 13.6 Hz), 4.1-4.2 (m, 2H), 4.35-4.45 (m, 1H), 4.45-4.55 (m, 1H), 4.73 (q, 2H, J = 2.3 Hz), 7.05 (d, 2H, J = 8.9 Hz), 7.81 (d, 2H, J = 8.9 Hz).

### (2) (3aS,6R,7S,7aR)-5-(4'-but-2'-ynyloxybenzenesulfonyl)-7-methoxy-2,2-dimethyl-hexahydro-[1,3]dioxolo[4,5-c]pyridine-6-carboxylic acid methyl ester:

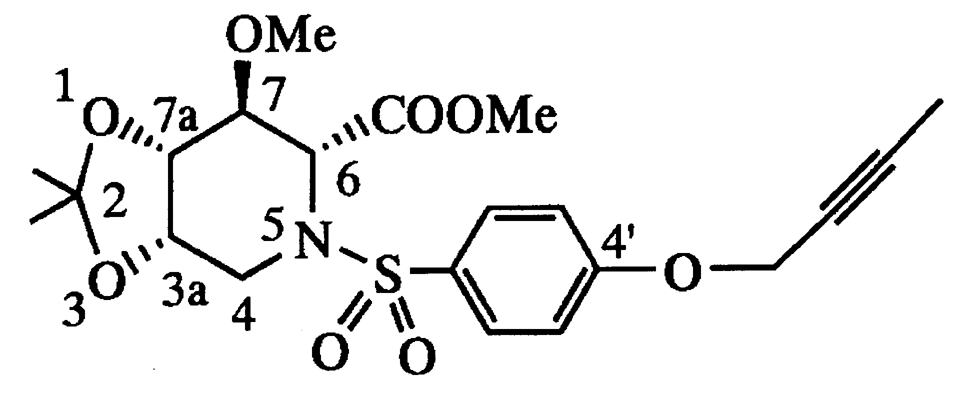

The compound (960 mg) of (1) was dissolved in methylene chloride (18 mL) and methyl iodide (3.1 g) and silver oxide (1.51 g) were added, followed by stirring under nitrogen atmosphere at room temperature for 5 days. The insoluble material was removed by filtration and the filtrate was concentrated under reduced pressure. The resulting residue was purified by silica gel medium pressure column chromatography (ethyl acetate:n-hexane = 1:3 → 1:2) to obtain the titled compound (775 mg) as a syrup.
¹H-NMR (CDCl₃) δ: 1.30 (s, 3H), 1.39 (s, 3H), 1.88 (t, 3H, J = 2.3 Hz), 3.26 (dd, 1H, J = 8.0, 13.3 Hz), 3.48 (s, 3H), 3.63 (dd, 1H, J = 6.6, 13.3 Hz), 3.69 (s, 3H), 4.1-4.2 (m, 2H), 4.25-4.35 (m, 1H), 4.72 (q, 2H, J = 2.3 Hz), 4.79 (d, 1H, J = 3.0 Hz), 7.05 (d, 2H, J = 9.0 Hz), 7.86 (d, 2H, J = 9.0 Hz).

### (3) (3aS,6R,7S,7aR)-5-(4'-but-2'-ynyloxybenzenesulfonyl)-7-methoxy-2,2-dimethyl-hexahydro-[1,3]dioxolo[4,5-c]pyridine-6-carboxylic acid hydroxamide:

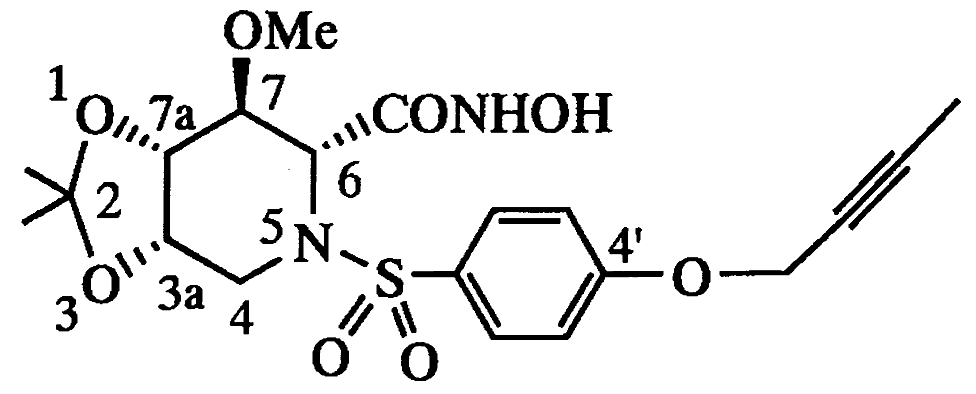

The compound (730 mg) of (2) was dissolved in methanol (12 mL) and an aqueous 50% hydroxylamine solution (3 mL) and sodium cyanide (79 mg) were added, followed by stirring at room temperature for 4 days. The reaction solution was distilled off under reduced pressure and the resulting residue was purified by silica gel medium pressure column chromatography (chloroform:methanol = 60:1 - 50:1 → 40:1) and then freeze-dried to obtain the titled compound (356 mg) as an amorphous.
¹H-NMR (DMSO-d₆) δ: 1.18 (s, 3H), 1.36 (s, 3H), 1.84 (s, 3H), 3.25 (s, 3H), 3.2-3.35 (m, 1H), 3.6-3.75 (m, 3H), 3.76 (d, 1H, J = 7.1 Hz), 4.05-4.15 (m, 1H), 4.87 (d, 2H, J = 2.2 Hz), 7.12 (d, 2H, J = 8.9 Hz), 7.75 (d, 2H, J = 8.9 Hz), 9.05 (s, 1H), 10.61 (s, 1H).
TOF-Mass: 477 (M+Na), 493 (M+K)
[α]_{D} 36°(c = 0.1, MeOH)

### (4) (2R,3S,4S,5S)-1-(4'-but-2'-ynyloxybenzenesulfonyl)-4,5-dihydroxy-3-methoxypiperidine-2-carboxylic acid hydroxamide:

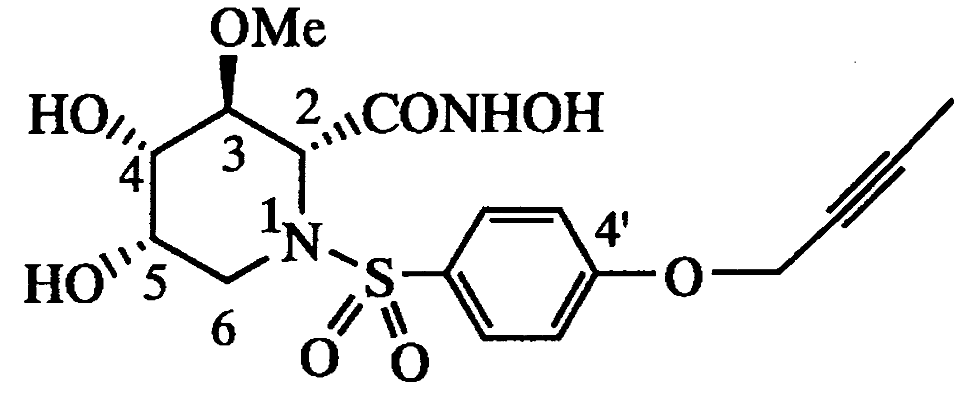

The compound (270 mg) of (3) was dissolved in methanol (10 mL) and a cation exchange resin (MUROMAC, 1.1 g) was added, followed by stirring at room temperature for 2 days. The insoluble material was removed by filtration and the filtrate was concentrated under reduced pressure. The resulting residue was purified by silica gel medium pressure column chromatography (chloroform:methanol = 50:1 → 30:1 → 10:1) and then freeze-dried to obtain the titled compound (111 mg) as an amorphous.
¹H-NMR (DMSO-d₆) δ: 1.84 (t, 3H, J = 2.3 Hz), 3.16 (s, 3H), 3.61 (bs, 1H), 3.68 (bs, 1H), 4.37 (s, 1H), 4.74 (bs, 1H), 4.84 (q, 2H, J = 2.3 Hz), 4.91 (bs, 1H), 7.05 (d, 2H, J = 8.9 Hz), 7.72 (d, 2H, J = 8.9 Hz), 8.87 (bs, 1H), 10.63 (s, 1H).
TOF-Mass: 437 (M+Na), 453 (M+K)
[α]_{D} 45°(c = 0.1, MeOH)

### Example 3

### Preparation of (2R,3S,4S,5S)-3-benzyloxy-1-(4'-but-2'-ynyloxybenzenesulfonyl)-4,5-dihydroxypiperidine-2-carboxylic acid hydroxamide:

### (1) (3aS,6R,7S,7aR)-7-benzyloxy-5-(4'-but-2'-ynyloxybenzenesulfonyl)-2,2-dimethyl-hexahydro-[1,3]dioxolo[4,5-c]pyridine-6-carboxylic acid methyl ester:

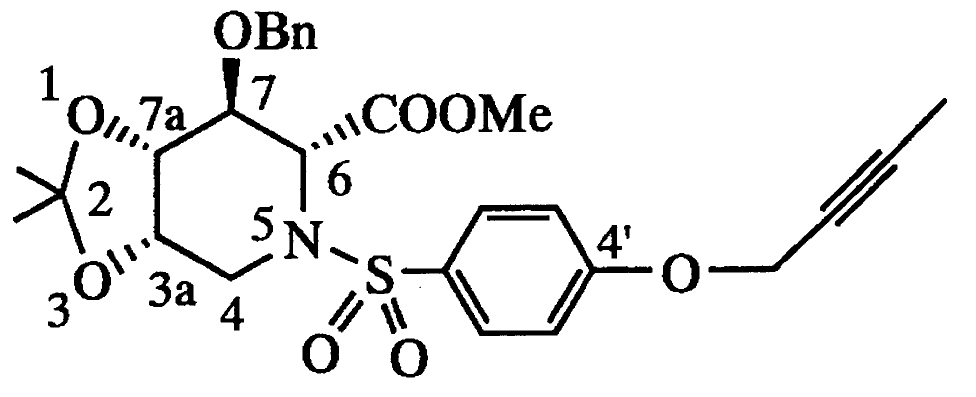

The compound (1.59 g) of Example 2 (1) was dissolved in methylene chloride (15 mL) and benzyl bromide (1.86 g) and silver oxide (3.34 g) were added, followed by stirring under nitrogen atmosphere at room temperature for 6 days. The insoluble material was removed by filtration and the filtrate was concentrated under reduced pressure. The resulting residue was purified by silica gel medium pressure column chromatography (ethyl acetate:n-hexane = 1:3 → 1:2) to obtain the titled compound (1.75 g) as a syrup.
¹H-NMR (CDCl₃)δ: 1.29 (s, 3H), 1.39 (s, 3H), 1.87 (t, 3H, J = 2.3 Hz), 3.32 (dd, 1H, J = 7.7, 13.3 Hz), 3.63 (dd, 1H, J = 6.3, 13.3 Hz), 3.68 (s, 3H), 4.19 (dd, 1H, J = 3.4, 5.9 Hz), 4.25-4.35 (m, 1H), 4.37 (t, 1H, J = 3.3 Hz), 4.58 (d, 1H, J = 11.4 Hz), 4.68 (q, 2H, J = 2.3 Hz), 4.76 (d, 1H, J = 11.4 Hz), 4.85 (d, 1H, J = 3.2 Hz), 6.97 (d, 2H, J = 9.0 Hz), 7.3-7.4 (m, 5H), 7.85 (d, 2H, J = 9.0 Hz).

### (2) (3aS,6R,7S,7aR)-7-benzyloxy-5-(4'-but-2'-ynyloxybenzenesulfonyl)-2,2-dimethyl-hexahydro-[1,3]dioxolo[4,5-c]pyridine-6-carboxylic acid hydroxamide:

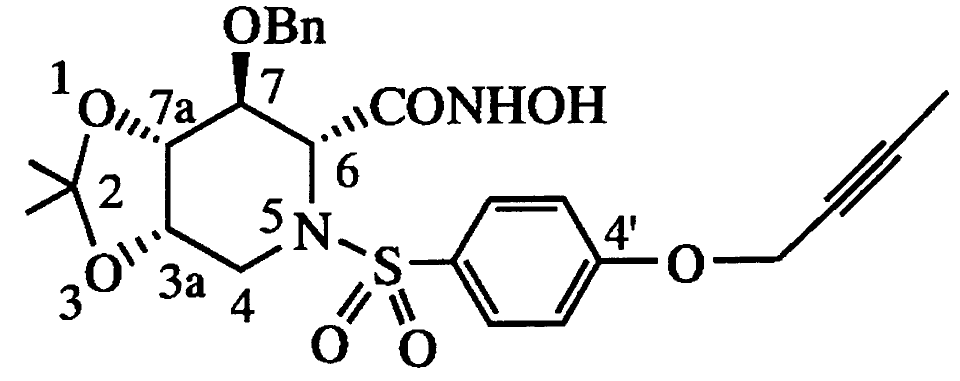

The compound (1.75 g) of (1) was dissolved in methanol (25 mL) and an aqueous 50% hydroxylamine solution (5 mL) and sodium cyanide (162 mg) were added, followed by stirring at room temperature for 3 days. The reaction solution was distilled off under reduced pressure and the resulting residue was purified by silica gel medium pressure column chromatography (chloroform:methanol = 30:1) and then freeze-dried to obtain the titled compound (247 mg) as an amorphous, and thus a raw material (252 mg) was recovered.
¹H-NMR (DMSO-d₆)δ: 1.20 (s, 3H), 1.36 (s, 3H), 1.81 (t, 3H, J = 2.3 Hz), 3.63 (dd, 1H, J = 5.7, 13.6 Hz), 3.75-3.85 (m, 1H), 3.9-3.95 (m, 2H), 4.15-4.25 (m, 1H), 4.43 (d, 1H, J = 11.5 Hz), 4.56 (d, 1H, J = 11.5 Hz), 4.83 (q, 2H, J = 2.3 Hz), 7.11 (d, 2H, J = 8.9 Hz), 7.2-7.4 (m, 5H), 7.83 (d, 2H, J = 8.9 Hz), 9.08 (s, 1H), 10.56 (s, 1H).
TOF-Mass: 553 (M+Na), 569 (M+K)
[α]_{D} 13°(c = 0.1, MeOH)

### (3) (2R,3S,4S,5S)-3-benzyloxy-1-(4'-but-2'-ynyloxybenzenesulfonyl)-4,5-dihydroxypiperidine-2-carboxylic acid hydroxamide:

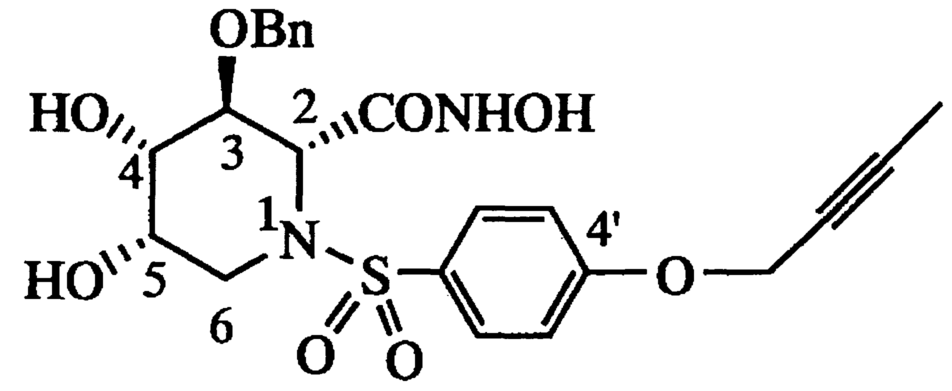

The compound (180 mg) of (2) was dissolved in methanol (15 mL) and a cation exchange resin (MUROMAC, 1 g) was added, followed by stirring at room temperature overnight and further stirring at 50°C for 3 hours. The insoluble material was removed by filtration and the filtrate was concentrated under reduced pressure. The resulting residue was purified by silica gel medium pressure column chromatography (chloroform:methanol = 50:1 → 40:1 → 30:1 → 20:1 → 10:1) and then freeze-dried to obtain the titled compound (59 mg) as an amorphous.
¹H-NMR (DMSO-d₆) δ: 1.82 (s, 3H), 3.67 (s, 1H), 3.92 (s, 1H), 4.48 (d, 1H, J = 12.0 Hz), 4.56 (d, 1H, J = 12.0 Hz), 4.77 (bs, 2H), 6.92 (d, 2H, J = 8.9 Hz), 7.2-7.4 (m, 5H), 7.71 (d, 2H, J = 8.9 Hz), 8.91 (s, 1H), 10.65 (s, 1H).
TOF-Mass: 513 (M+Na), 529 (M+K)
[α]_{D} 11°(c = 0.1, MeOH)

### Example 4

### Preparation of (2R,3S,4R,5S)-1-(4'-but-2'-ynyloxybenzenesulfonyl)-3,4-dihydroxy-5-methoxypiperidine-2-carboxylic acid hydroxamide:

### (1) (3aS,4R,7S,7aS)-5-(4'-but-2'-ynyloxybenzenesulfonyl)-7-methoxy-2,2-dimethyl-hexahydro-[1,3]dioxolo[4,5-c]pyridine-4-carboxylic acid methyl ester:

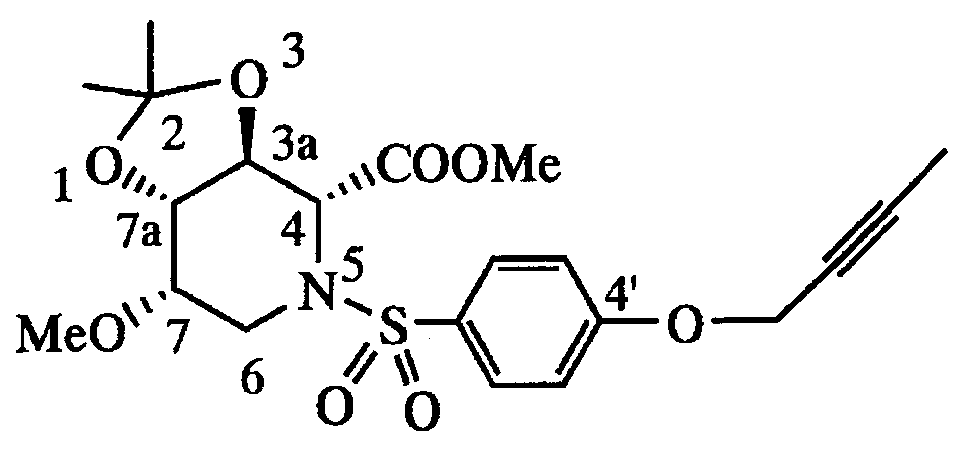

The compound (1.0 g) of Example 1 was dissolved in methylene chloride (15 mL) and methyl iodide (3.2 g) and silver oxide (1.58 g) were added, followed by stirring under nitrogen atmosphere at room temperature for 4 days. The insoluble material was removed by filtration and the filtrate was concentrated under reduced pressure. The resulting residue was purified by silica gel medium pressure column chromatography (ethyl acetate:cyclohexane = 1:3 → 1:2) to obtain the titled compound (852 mg) as a syrup.
¹H-NMR (CDCl₃)δ: 1.44 (s, 3H), 1.47 (s, 3H), 1.88 (t, 3H, J = 2.3 Hz), 3.38 (dd, 1H, J = 5.1, 14.3 Hz), 3.45 (s, 3H), 3.5-3.6 (m, 3H), 3.79 (s, 3H), 3.95-4.0 (m, 2H), 4.73 (q, 2H, J = 2.3 Hz), 7.08 (d, 2H, J = 9.0 Hz), 7.83 (d, 2H, J = 9.0 Hz).

### (2) (3aS,4R,7S,7aS)-5-(4'-but-2'-ynyloxybenzenesulfonyl)-7-methoxy-2,2-dimethyl-hexahydro-[1,3]dioxolo[4,5-c]pyridine-4-carboxylic acid hydroxamide:

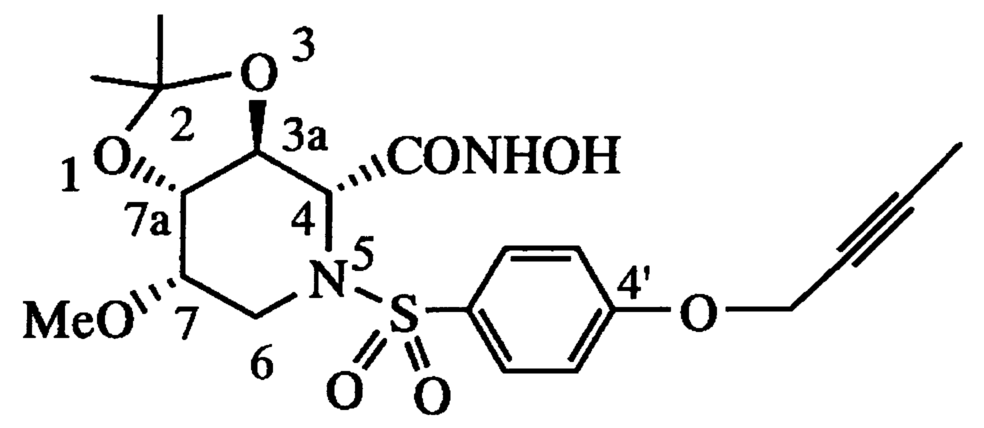

The compound (852 mg) of (1) was dissolved in methanol (20 mL) and an aqueous 50% hydroxylamine solution (5 mL) and sodium cyanide (92 mg) were added, followed by stirring at room temperature overnight. The reaction solution was distilled off under reduced pressure and the resulting residue was purified by silica gel medium pressure column chromatography (chloroform:methanol = 30:1) and then freeze-dried to obtain the titled compound (730 mg) as an amorphous.
¹H-NMR (DMSO-d₆)δ: 1.23 (s, 3H), 1.28 (s, 3H), 1.84 (t, 3H, J = 2.2 Hz), 2.96 (dd, 1H, J = 4.0, 10.0 Hz), 3.25-3.45 (m, 2H), 3.72 (d, 1H, J = 8.7 Hz), 3.75 (d, 1H, J = 6.5 Hz), 4.02 (t, 1H, J = 9.5 Hz), 4.88 (t, 2H, J = 2.2 Hz), 7.17 (d, 2H, J = 8.9 Hz), 7.83 (d, 2H, J = 8.9 Hz), 9.10 (s, 1H), 10.93 (s, 1H).
TOF-Mass: 477 (M+Na), 493 (M+K)
[α]_{D} 33° (c = 0.1, MeOH)

### (3) (2R, 3S, 4R, 5S)-1-(4'-but-2'-ynyloxybenzenesulfonyl)-3,4-dihydroxy-5-methoxypiperidine-2-carboxylic acid hydroxamide:

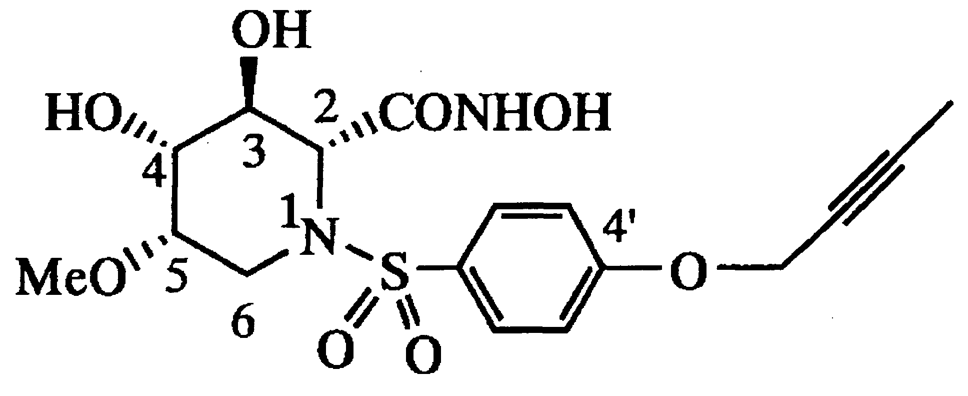

The compound (500 mg) of (2) was dissolved in methanol (15 mL) and a cation exchange resin (MUROMAC, 2 g) was added, followed by stirring at room temperature overnight. The insoluble material was removed by filtration and the filtrate was concentrated under reduced pressure. The resulting residue was purified by silica gel medium pressure column chromatography (chloroform:methanol = 50:1 → 40:1 → 30:1 → 20:1 → 10:1) and then freeze-dried to obtain the titled compound (370 mg) as an amorphous.
¹H-NMR (DMSO-d₆)δ: 1.84 (t, 3H, J = 2.2 Hz), 3.14 (s, 3H), 3.2-3.5 (m, 4H), 3.48 (s, 1H), 3.70 (s, 1H), 3.78 (s, 1H), 4.84 (bs, 2H), 4.91 (bs, 1H), 5.29 (bs, 1H), 7.08 (d, 2H, J = 8.9 Hz), 7.79 (d, 2H, J = 8.9 Hz), 8.83 (s, 1H), 10.55 (s, 1H).
TOF-Mass: 437 (M+Na), 453 (M+K)
[α]_{D} -4°(c = 0.1, MeOH)

### Example 5

### Preparation of (2R,3S,4S,5S)-1-(4'-but-2'-ynyloxybenzenesulfonyl)-3,4,5-trihydroxy-piperidine-2-carboxylic acid hydroxamide:

### (1) (3aS,4R,7S,7aS)-5-(4'-but-2'-ynyloxybenzenesulfonyl)-7-hydroxy-2,2-dimethyl-hexahydro-[1,3]dioxolo[4,5-c]pyridine-4-carboxylic acid hydroxamide:

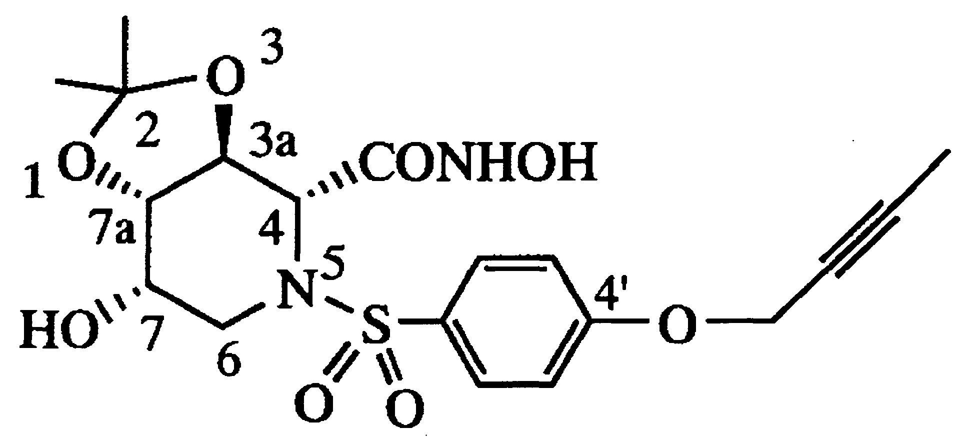

The compound (480 mg) of Example 1 was dissolved in methanol (8 mL) and an aqueous 50% hydroxylamine solution (3 mL) and sodium cyanide (54 mg) were added, followed by stirring at room temperature overnight. The reaction solution was distilled off under reduced pressure and the resulting residue was purified by silica gel medium pressure column chromatography (chloroform:methanol = 50:1 → 40:1 → 30:1) and then freeze-dried to obtain the titled compound (210 mg) as an amorphous.
¹H-NMR (DMSO-d₆) δ: 1.22 (s, 3H), 1.28 (s, 3H), 1.83 (t, 3H, J = 2.1 Hz), 2.76 (dd, 1H, J = 4.5, 10.0 Hz), 3.19 (dd, 1H, J = 6.0, 14.6 Hz), 3.74 (d, 1H, J = 8.9 Hz), 3.80 (dd, 1H, J = 6.9, 14.5 Hz), 4.0-4.1 (m, 2H), 4.8-4.95 (m, 2H), 5.2 (d, 1H, J = 3.7 Hz), 7.16 (d, 2H, J = 8.9 Hz), 7.84 (d, 2H, J = 8.9 Hz), 9.09 (s, 1H), 10.93 (s, 1H).
TOF-Mass: 463 (M+Na), 479 (M+K)
[α]_{D} 52°(c = 0.1, MeOH)

### (2) (2R,3S,4S,5S)-1-(4'-but-2'-ynyloxybenzenesulfonyl)-3,4,5-trihydroxy-piperidine-2-carboxylic acid hydroxamide:

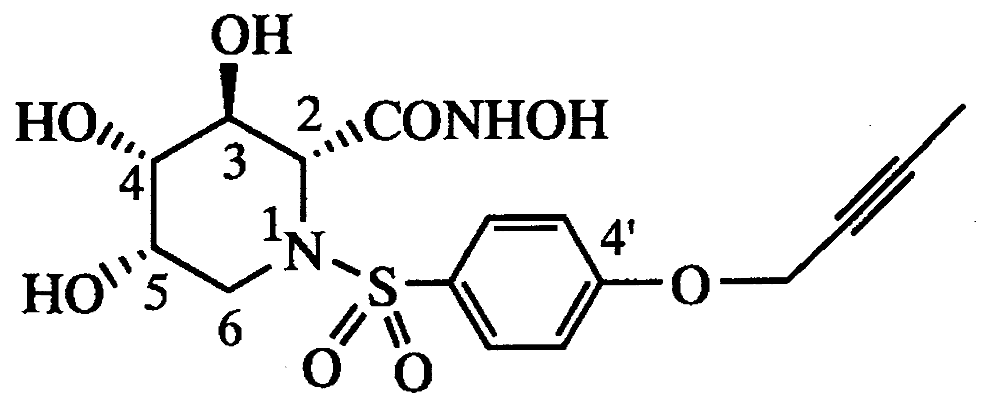

The compound (156 mg) of (1) was dissolved in methanol (8 mL) and a cation exchange resin (MUROMAC, 1 g) was added, followed by stirring at room temperature overnight. The insoluble material was removed by filtration and the filtrate was concentrated under reduced pressure. The resulting residue was purified by silica gel medium pressure column chromatography (chloroform:methanol = 30:1 → 10:1 → 5:1) and then freeze-dried to obtain the titled compound (120 mg) as an amorphous.
¹H-NMR (DMSO-d₆) δ: 1.84 (s, 3H), 3.19 (dd, 1H, J = 4.5, 12.8 Hz), 3.4-3.6 (m, 2H), 4.05 (s, 1H), 4.26 (s, 1H), 4.7 (bs, 1H), 4.83 (s, 2H), 5.27 (s, 1H), 7.07 (d, 2H, J = 8.7 Hz), 7.80 (d, 2H, J = 8.7 Hz), 8.86 (bs, 1H), 10.59 (s, 1H).
TOF-Mass: 423 (M+Na), 439 (M+K)
[α]_{D} 3°(c = 0.1, MeOH)

### Example 6

### Preparation of (3aS,6R,7S,7aR)-5-(4'-but-2'-ynyloxybenzenesulfonyl)-7-hydroxy-2,2-dimethyl-hexahydro-[1,3]dioxolo[4,5-c]pyridine-6-carboxylic acid hydroxamide:

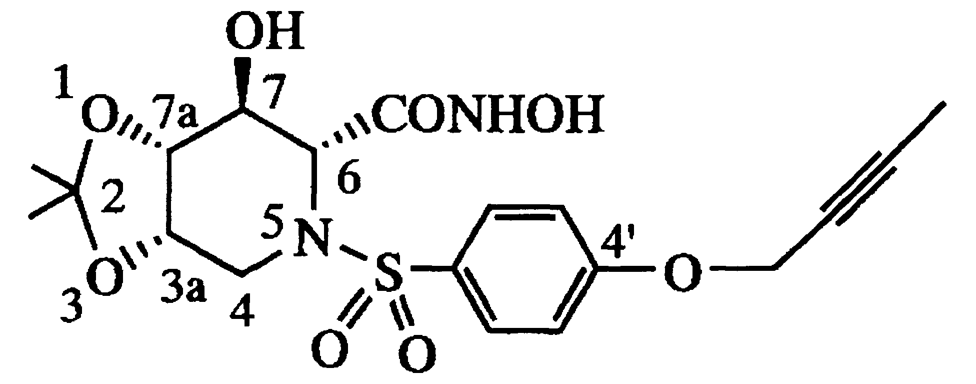

The compound (642 mg) of Example 2 (1) was dissolved in methanol (30 mL) and an aqueous 50% hydroxylamine solution (6 mL) and sodium cyanide (72 mg) were added, followed by stirring at room temperature overnight. The reaction solution was distilled off under reduced pressure and the resulting residue was purified by silica gel medium pressure column chromatography (chloroform:methanol = 50:1 → 30:1) and then freeze-dried to obtain the titled compound (350 mg) as an amorphous.
¹H-NMR (DMSO-d₆) δ: 1.17 (s, 3H), 1.34 (s, 3H), 1.83 (s, 3H), 3.16 (d, 1H, J = 8.2 Hz), 3.74 (dd, 1H, J = 6.3, 13.8 Hz), 3.83 (dd, 1H, J = 7.9, 13.5 Hz), 4.0-4.1 (m, 1H), 4.8-4.9 (m, 2H), 5.76 (d, 1H, J = 5.5 Hz), 7.14 (d, 2H, J = 8.9 Hz), 7.83 (d, 2H, J = 8.9 Hz), 8.98 (s, 1H), 10.56 (s, 1H).
TOF-Mass: 463 (M+Na), 479 (M+K)
[α]_{D} 50°(c = 0.1, MeOH)

### Example 7

### Preparation of (2R,3S,4S,5S)-3,4,5-trihydroxy-1-[4'-(4"-hydroxy-but-2'-ynyloxy)benzenesulfonyl]-piperidine-2-carboxylic acid hydroxamide:

### (1) (3aS, 4R, 7S,7aS)-5-[4'-[4"-(tert-butyldimethylsilanyloxy)-but-2'-ynyloxy]benzenesulfonyl]-7-hydroxy-2,2-dimethylhexahydro-[1,3]dioxolo[4,5-c]pyridine-4-carboxylic acid methyl ester:

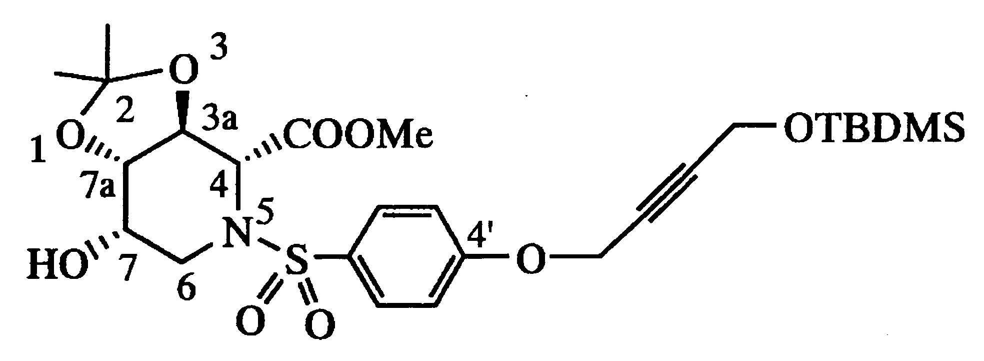

The compound (767 mg) of Example 1 (4) was dissolved in ethyl acetate (20 mL) and 10% Pd/C (92 mg) was added, followed by stirring under hydrogen atmosphere at 40°C for 2.5 hours. The catalyst was removed by filtration and the filtrate was concentrated under reduced pressure. The resulting residue was dissolved in methylene chloride (12 mL) and (4-bromo-but-2-ynyloxy)-tert-butyldimethylsilane (550 mg) and silver oxide (484 mg) were added, followed by stirring under nitrogen atmosphere overnight. Furthermore, (4-bromo-but-2-ynyloxy)-tert-butyldimethylsilane (366 mg) and silver oxide (322 mg) were added, followed by stirring at 50°C for 3 hours. The insoluble material was removed by filtration and the filtrate was concentrated under reduced pressure. The resulting residue was purified by silica gel medium pressure column chromatography (ethyl acetate:cyclohexane = 1:3 → 2:3) to obtain the titled compound (393 mg) as a syrup.
¹H-NMR (CDCl₃)δ: 0.09 (s, 6H), 0.89 (s, 9H), 1.45 (s, 3H), 1.47 (s, 3H), 2.26 (s, 1H), 3.22 (dd, 1H, J = 5.7, 14.7 Hz), 3.49 (dd, 1H, J = 4.3, 9.7 Hz), 3.75 (s, 3H), 3.85 (dd, 1H, J = 7.1, 14.7Hz), 4.05-4.15 (m, 1H), 4.35 (s, 2H), 4.37-4.42 (m, 1H), 4.43 (d, 1H, J = 9.0 Hz), 4.80 (s, 2H), 7.06 (d, 2H, J = 8.9 Hz), 7.81 (d, 2H, J = 8.9 Hz).
TOF-Mass: 592 (M+Na), 608 (M+K)

### (2) (3aS,4R,7S,7aS)-5-[4'-[4"-(tert-butyldimethylsilanyloxy)-but-2'-ynyloxy]benzenesulfonyl]-7-hydroxy-2,2-dimethylhexahydro-[1,3]dioxolo[4,5-c]pyridine-4-carboxylic acid hydroxamide:

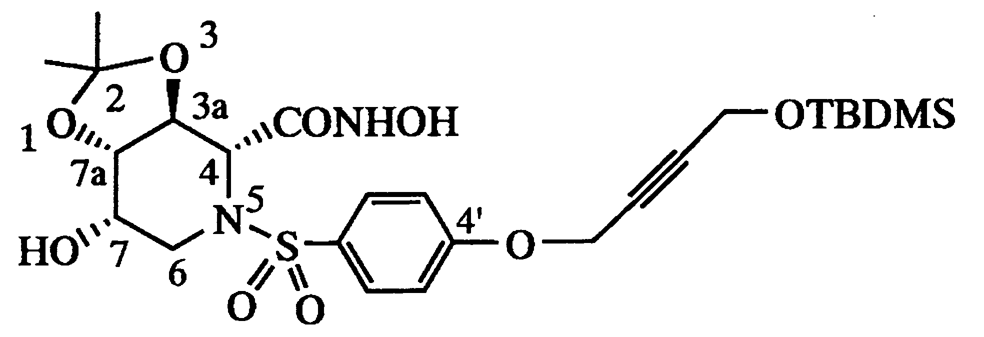

The compound (378 mg) of (1) was dissolved in methanol (15 mL) and an aqueous 50% hydroxylamine solution (3 mL) and sodium cyanide (32 mg) were added, followed by stirring at room temperature overnight. The reaction solution was distilled off under reduced pressure and the resulting residue was purified by silica gel medium pressure column chromatography (chloroform:methanol = 50:1 → 30:1) and then freeze-dried to obtain the titled compound (227 mg) as an amorphous.
¹H-NMR (DMSO-d₆) δ: 0.04 (s, 6H), 0.84 (s, 9H), 1.22 (s, 3H), 1.28 (s, 3H), 2.78 (dd, 1H, J = 4.4, 9.6 Hz), 3.19 (dd, 1H, J = 5.8, 14.5 Hz), 3.34 (s, 3H), 3.7-3.8 (m, 2H), 4.0-4.1 (m, 2H), 4.35 (s, 2H), 4.99 (s, 2H), 5.23 (d, 1H, J = 4.6 Hz), 7.17 (d, 2H, J = 8.9 Hz), 7.82 (d, 2H, J = 8.9 Hz), 9.11 (s, 1H), 10.92 (s, 1H).
TOF-Mass: 593 (M+Na), 609 (M+K)

### (3) (3aS,4R,7S,7aS)-7-hydroxy-5-[4'-(4"-hydroxy-but-2'-ynyloxy)benzenesulfonyl]-2,2-dimethyl-hexahydro-[1,3]dioxolo[4,5-c]pyridine-4-carboxylic acid hydroxamide:

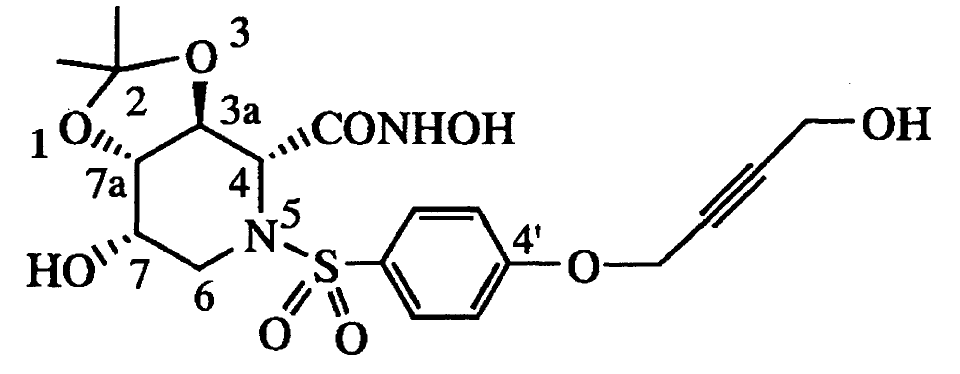

The compound (217 mg) of (2) was dissolved in THF (5 mL) and acetic acid (62 mg) was added, followed by the addition of TBAF (1 mL) and further stirring at room temperature for 3 hours. The reaction solution was distilled off under reduced pressure and the resulting residue was purified by silica gel medium pressure column chromatography (chloroform:methanol = 50:1 → 25:1 → 10:1) and then freeze-dried to obtain the titled compound (151 mg) as an amorphous. ¹H-NMR (DMSO-d₆) δ: 1.22 (s, 3H), 1.28 (s, 3H), 2.79 (dd, 1H, J = 4.4, 10.0 Hz), 3.19 (dd, 1H, J = 5.8, 14.5 Hz), 3.7-3.85 (m, 2H), 4.0-4.1 (m, 2H), 4.10 (d, 1H, J = 5.9 Hz), 4.97 (s, 2H), 5.23 (d, 1H, J = 4.5 Hz), 5.27 (t, 1H, J = 5.9 Hz), 7.17 (d, 2H, J = 8.9 Hz), 7.83 (d, 2H, J = 8.9 Hz), 9.11 (s, 1H), 10.92 (s, 1H).
TOF-Mass: 479 (M+Na), 495 (M+K)

### (4) (2R,3S,4S,5S)-3,4,5-trihydroxy-1-[4'-(4"hydroxy-but-2'-ynyloxy)benzenesulfonyl]-piperidine-2-carboxylic acid hydroxamide:

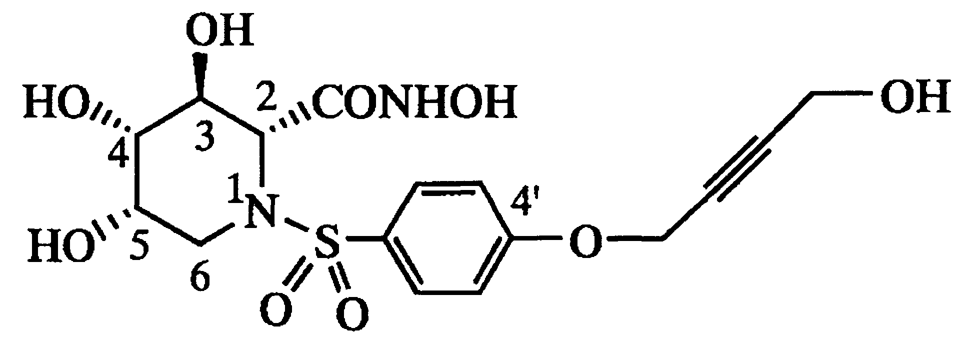

The compound (120 mg) of (3) was dissolved in methanol (10 mL) and a cation exchange resin (MUROMAC, 2.4 g) was added, followed by stirring at room temperature overnight. The insoluble material was removed by filtration and the filtrate was concentrated under reduced pressure. The resulting residue was purified by silica gel medium pressure column chromatography (chloroform:methanol = 20:1 → 5:1 → 4:1) and then freeze-dried to obtain the titled compound (26 mg) as an amorphous.
¹H-NMR (DMSO-d₆)δ: 3.19 (dd, 1H, J = 4.7, 12.4 Hz), 3.25-3.35 (m, 1H), 3.4-3.6 (m, 2H), 4.05 (bs, 1H), 4.12 (d, 1H, J = 5.9 Hz), 4.26 (s, 1H), 4.67 (d, 1H, J = 6.4 Hz), 4.89 (bs, 1H), 4.93 (s, 1H), 5.25-5.35 (m, 2H), 7.09 (d, 2H, J = 8.8 Hz), 7.79 (d, 2H, J = 8.8 Hz), 8.86 (s, 1H), 10.59 (bs, 1H).
TOF-Mass: (M+Na), (M+K)

### Example 8

### Preparation of (2R,3S,4S,5S)-1-(4'-but-2'-ynyloxybenzenesulfonyl)-4,5-dihydroxy-3-isobutoxypiperidine-2-carboxylic acid hydroxamide:

### (1) (3aS,6R,7S,7aR)-5-(4'-benzyloxybenzenesulfonyl)-7-hydroxy-2,2-dimethyl-hexahydro-[1,3]dioxolo[4,5-c]pyridine-6-carboxylic acid methyl ester:

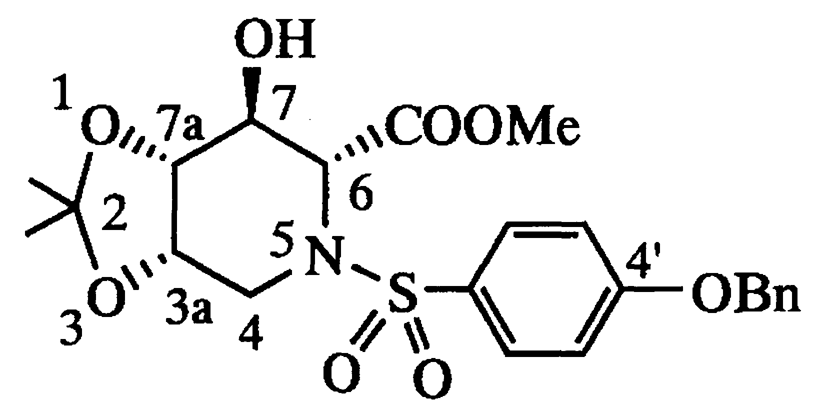

The compound (4.8 g) of Example 1 (4) was dissolved in methanol (70 mL) and a cation exchange resin (MUROMAC, 10.4 g) was added, followed by stirring at room temperature overnight. The insoluble material was removed by filtration and the filtrate was concentrated under reduced pressure. The resulting residue was dissolved in DMF (40 mL) and DMP (10.7 mL) and p-toluenesulfonic acid monohydrate (84 mg) were added, followed by stirring at room temperature overnight. The reaction solution was mixed with ethyl acetate (100 mL) and then washed with an aqueous saturated sodium hydrogencarbonate solution and saturated saline. After drying over magnesium sulfate, the solvent was distilled off under reduced pressure. The resulting residue was purified by silica gel medium pressure column chromatography (ethyl acetate:cyclohexane = 2:3) to obtain the titled compound (3.62 g) as a syrup.
¹H-NMR (CDCl₃)δ: 1.27 (s, 3H), 1.37 (s, 3H), 2.92 (bs, 1H), 3.21 (dd, 1H, J = 8.9, 13.5 Hz), 3.62 (s, 3H), 3.80 (dd, 1H, J = 7.1, 13.4 Hz), 4.05-4.15 (m, 1H), 4.38 (dt, 1H, J = 7.0, 7.8 Hz), 4.52 (bs, 1H), 4.58 (d, 1H, J = 4.1 Hz), 7.04 (d, 2H, J = 8.9 Hz), 7.30-7.45 (m, 5H), 7.80 (d, 2H, J = 8.9 Hz).

### (2) (3aS, 6R, 7S, 7aR) -5- (4'-benzyloxybenzenesulfonyl)-2,2-dimethyl-7-(2"-methylallyloxy)-hexahydro-[1,3]dioxolo[4,5-c]pyridine-6-carboxylic acid methyl ester:

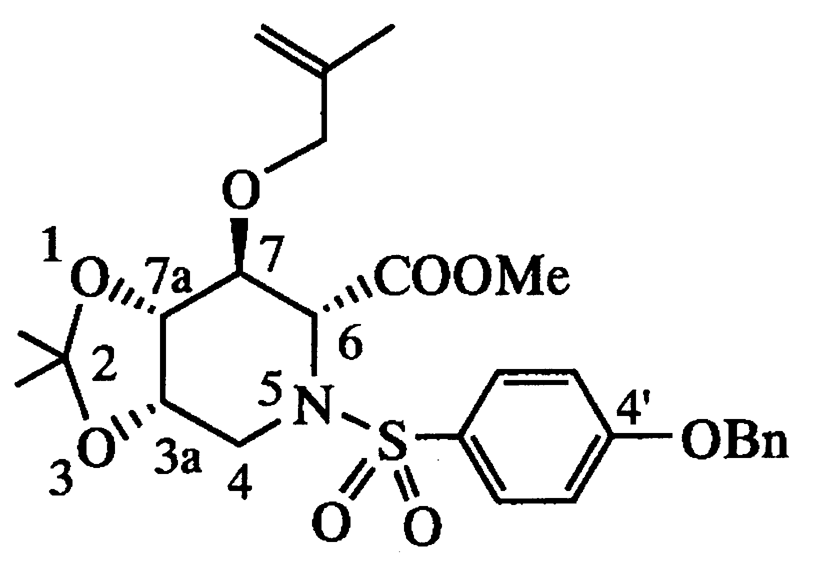

The compound (800 mg) of (1) was dissolved in methylene chloride (12 mL) and 3-bromo-2-methylpentene (1.13 g) and silver oxide (970 mg) were added, followed by stirring under nitrogen atmosphere at room temperature for 4 days. The insoluble material was removed by filtration and the filtrate was concentrated under reduced pressure. The resulting residue was purified by silica gel medium pressure column chromatography (ethyl acetate:n-hexane = 1:4 → 1:3) to obtain the titled compound (754 mg) as a syrup.
¹H-NMR (CDCl₃)δ: 1.31 (s, 3H), 1.39 (s, 3H), 1.76 (s, 3H), 3.29 (dd, 1H, J = 7.6, 13.6 Hz), 3.61 (dd, 1H, J = 6.3, 13.1 Hz), 3.97 (d, 1H, J = 12.2 Hz), 4.09 (d, 1H, J = 12.2 Hz), 4.18 (dd, 1H, J = 3.3, 5.9 Hz), 4.25-4.35 (m, 2H), 4.76 (d, 1H, J = 3.1 Hz), 4.95 (s, 1H), 4.99 (s, 1H), 5.13 (s, 1H), 7.04 (d, 2H, J = 8.9 Hz), 7.3-7.5 (m, 5H), 7.86 (d, 2H, J = 8.9 Hz).

### (3) (3aS,6R,75,7aR)-5-(4'-but-2'-ynyloxybenzenesulfonyl)-7-isobutoxy-2,2-dimethyl-hexahydro-[1,3]dioxolo[4,5-c]pyridine-6-carboxylic acid methyl ester:

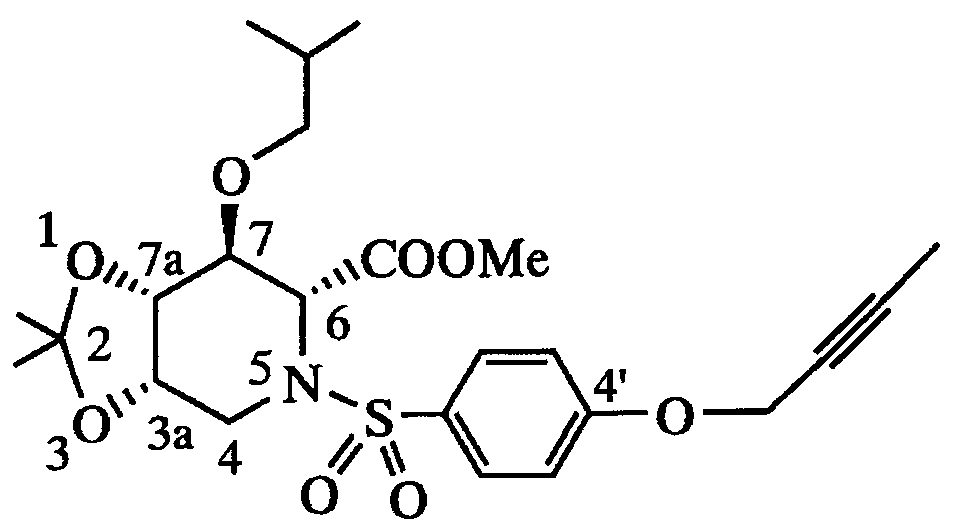

The compound (750 mg) of (2) was dissolved in ethyl acetate (15 mL) and 10% Pd/C (100 mg) was added, followed by stirring under hydrogen atmosphere at room temperature for 3 hours. The catalyst was removed by filtration and the filtrate was concentrated under reduced pressure. The resulting residue was dissolved in methylene chloride (15 mL) and 1-bromo-2-butyne (279 mg) and silver oxide (487 mg) were added, followed by stirring under nitrogen atmosphere overnight. The insoluble material was removed by filtration and the filtrate was concentrated under reduced pressure. The resulting residue was purified by silica gel medium pressure column chromatography (ethyl acetate:n-hexane = 1:4 → 1:3) to obtain the titled compound (502 mg) as a syrup.
¹H-NMR (CDCl₃)δ: 0.90 (d, 6H, J = 6.7 Hz), 1.31 (s, 3H), 1.40 (s, 3H), 1.75-1.85 (m, 1H), 1.88 (t, 3H, J = 2.0 Hz), 3.25-3.35 (m, 2H), 3.43 (dd, 1H, J = 6.6, 8.7 Hz), 3.57 (dd, 1H, J = 6.2, 13.2 Hz), 3.70 (s, 3H), 4.17 (dd, 1H, J = 3.3, 6.0 Hz), 4.22 (t, 1H, J = 3.2 Hz), 4.71 (q, 2H, J = 2 Hz), 4.73 (d, 1H, J = 3.1 Hz), 7.04 (d, 2H, J = 8.8 Hz), 7.88 (d, 2H, J = 8.8 Hz).

### (4) (3aS,6R,7S,7aR)-5-(4'-but-2'-ynyloxybenzenesulfonyl)-7-isobutoxy-2,2-dimethyl-hexahydro-[1,3]dioxolo[4,5-c]pyridine-6-carboxylic acid hydroxamide:

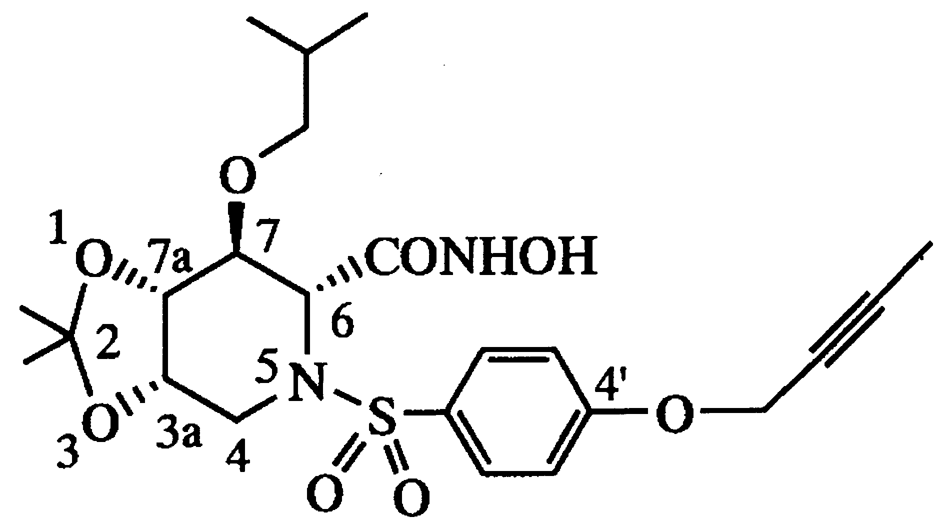

The compound (500 mg) of (3) was dissolved in methanol (15 mL) and an aqueous 50% hydroxylamine solution (3 mL) and sodium cyanide (50 mg) were added, followed by stirring at room temperature overnight. The reaction solution was distilled off under reduced pressure and the resulting residue was purified by silica gel medium pressure column chromatography (chloroform:methanol = 50:1 → 40:1 → 30:1) and then freeze-dried to obtain the titled compound (317 mg) as an amorphous.
¹H-NMR (DMSO-d₆) δ: 0.73 (d, 6H, J = 6.6 Hz), 1.19 (s, 3H), 1.36 (s, 3H), 1.45-1.6 (m, 1H), 1.83 (s, 3H), 3.0-3.1 (m, 1H), 3.2-3.35 (m, 2H), 3.60 (dd, 1H, J = 5.7, 13.7 Hz), 3.65-3.8 (m, 2H), 4.1-4.2 (m, 1H), 4.87 (s, 2H), 7.15 (d, 2H, J = 8.0 Hz), 7.82 (d, 2H, J = 8.0 Hz), 9.03 (s, 1H), 10.50 (s, 1H).

### (5) (2R,3S,4S,5S)-1-(4'-but-2'-ynyloxybenzenesulfonyl)-4,5-dihydroxy-3-isobutoxypiperidine-2-carboxylic acid hydroxamide:

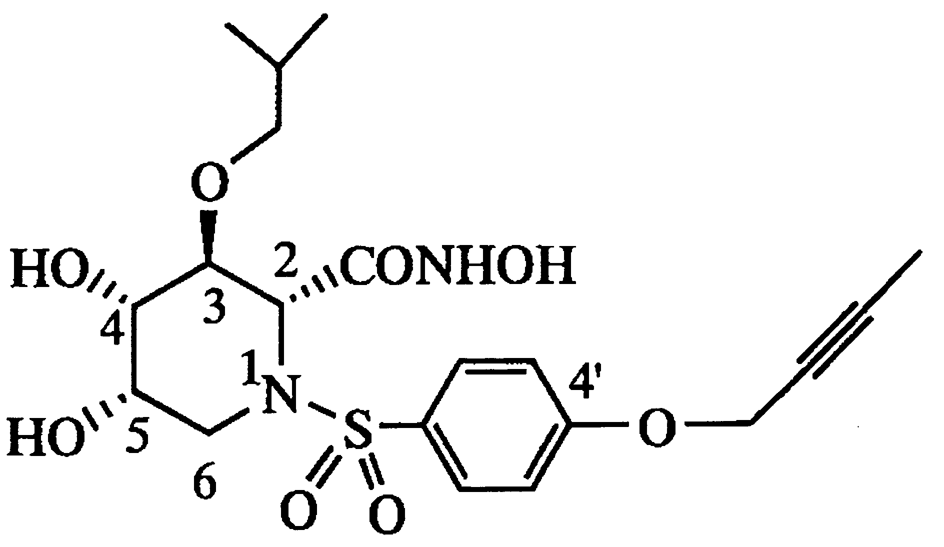

The compound (275 mg) of (4) was dissolved in methanol (10 mL) and a cation exchange resin (MUROMAC, 2 g) was added, followed by stirring at room temperature for 2 days. The insoluble material was removed by filtration and the filtrate was concentrated under reduced pressure. The resulting residue was purified by silica gel medium pressure column chromatography (chloroform:methanol = 50:1 → 40:1 → 30:1 → 10:1) and then freeze-dried to obtain the titled compound (49 mg) as an amorphous, and thus a raw material (157 mg) was recovered.
¹H-NMR (DMSO-d₆)δ: 0.75-0.85 (m, 6H), 1.6-1.7 (m, 1H), 1.84 (t, 1H, J = 2.3 Hz), 3.12 (dd, 1H, J = 7.0, 8.9 Hz), 3.24 (dd, 1H, J = 6.0, 9.1 Hz), 3.63 (bs, 1H), 3.75 (bs, 1H), 4.34 (bs, 1H), 4.79 (bs, 1H), 4.83 (q, 2H, J = 2.3 Hz), 4.92 (bs, 1H), 7.08 (d, 2H, J = 8.8 Hz), 7.74 (d, 2H, J = 8.8 Hz), 8.92 (s, 1H), 10.63 (s, 1H).

### Example 9

### Preparation of (2R,3S,4S,5S)-1-(4'-but-2'-ynyloxybenzenesulfonyl)-4,5-dihydroxy-3-ethoxypiperidine-2-carboxylic acid hydroxamide:

### (1) (3aS,6R,7S,7aR)-5-(4'-but-2'-ynyloxybenzenesulfonyl)-7-ethoxy-2,2-dimethyl-hexahydro-[1,3]dioxolo[4,5-c]pyridine-6-carboxylic acid methyl ester:

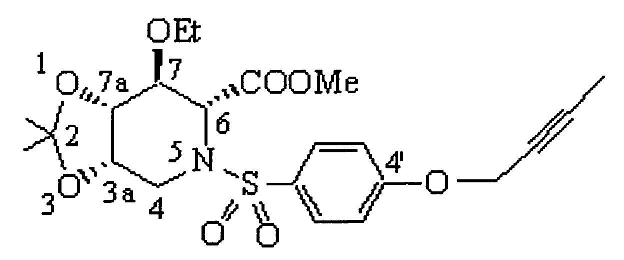

The compound (1.47 g) of Example 2 (1) was dissolved in methylene chloride (15 mL) and ethyl iodide (5.2 g) and silver oxide (2.3 g) were added, followed by stirring under nitrogen atmosphere at room temperature for 6 days. The insoluble material was removed by filtration and the filtrate was concentrated under reduced pressure. The resulting residue was purified by silica gel medium pressure column chromatography (ethyl acetate:n-hexane = 1:4 → 1:3) to obtain the titled compound (485 mg) as a syrup.
¹H-NMR (CDCl₃) δ: 1.19 (t, 3H, J = 7.0 Hz), 1.30 (s, 3H), 1.39 (s, 3H), 1.88 (t, 3H, J = 2.2 Hz), 3.30 (dd, 1H, J = 7.6, 13.3 Hz), 3.55-3.65 (m, 2H), 3.70 (s, 3H), 3.7-3.8 (m, 1H), 4.15-4.2 (m, 1H), 4.24 (t, 1H, J = 3.3 Hz), 4.29 (q, 1H, J = 6.2 Hz), 4.72 (q, 2H, J = 2.2 Hz), 4.73 (d, 1H, J = 3.2 Hz), 7.04 (d, 2H, J = 8.9 Hz), 7.86 (d, 2H, J = 8.9 Hz).

### (2) (3aS,6R,7S,7aR)-5-(4'-but-2'-ynyloxybenzenesulfonyl)-7-ethoxy-2,2-dimethyl-hexahydro-[1,3]dioxolo[4,5-c]pyridine-6-carboxylic acid hydroxamide:

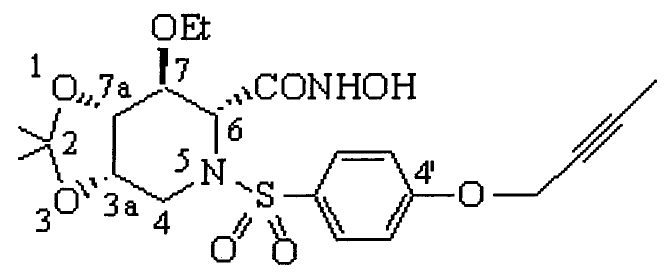

The compound (480 mg) of (1) was dissolved in methanol (15 mL) and an aqueous 50% hydroxylamine solution (3 mL) and sodium cyanide (50 mg) were added, followed by stirring at room temperature for 2 days. The reaction solution was distilled off under reduced pressure and the resulting residue was purified by silica gel medium pressure column chromatography (chloroform:methanol = 60:1 → 40:1 → 30:1) and then freeze-dried to obtain the titled compound (205 mg) as an amorphous.
¹H-NMR (DMSO-d₆) δ: 0.93 (t, 3H, J = 7.0 Hz), 1.18 (s, 3H), 1.36 (s, 3H), 1.83 (t, 3H, J = 1.7 Hz), 3.29 (dd, 1H, J = 8.3, 13.4 Hz), 3.5-3.55 (m, 1H), 3.6-3.75 (m, 4H), 4.12 (q, 1H, J = 7.7 Hz), 4.87 (q, 2H, J = 2.2 Hz), 7.15 (d, 2H, J = 8.9 Hz), 7.82 (d, 2H, J = 8.9 Hz), 9.04 (s, 1H), 10.54 (s, 1H).
TOF-Mass: 491 (M+Na), 500 (M+K)

### (3) (2R,3S,4S,5S)-1-(4'-but-2'-ynyloxybenzenesulfonyl)-4,5-dihydroxy-3-ethoxypiperidine-2-carboxylic acid hydroxamide:

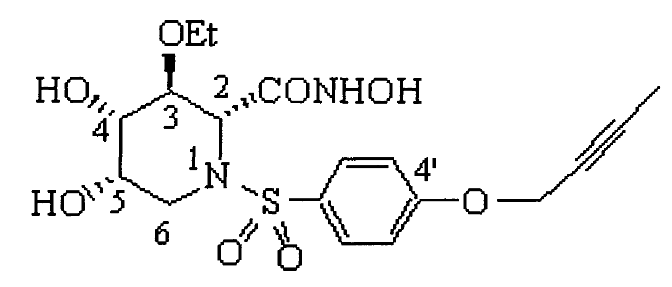

The compound (165 mg) of (2) was dissolved in methanol (15 mL) and a cation exchange resin (MUROMAC, 2.5 g) was added, followed by stirring at room temperature for 2 days. The insoluble material was removed by filtration and the filtrate was concentrated under reduced pressure. The resulting residue was purified by silica gel medium pressure column chromatography (chloroform:methanol = 50:1 → 30:1 → 10:1) and then freeze-dried to obtain the titled compound (44 mg) as an amorphous.
¹H-NMR (DMSO-d₆) δ: 1.01 (t, 3H, J = 6.0 Hz), 1.84 (t, 3H, J = 2.3 Hz), 3.45-3.55 (m, 1H), 3.59 (bs, 1H), 3.78 (bs, 1H), 4.35 (bs, 1H), 4.75 (bs, 1H), 4.84 (q, 2H, J = 2.3 Hz), 4.91 (bs, 1H), 7.07 (d, 2H, J = 8.7 Hz), 7.74 (d, 2H, J = 8.7 Hz), 8.88 (s, 1H), 10.62 (s, 1H).
TOF-Mass: 451 (M+Na), 467 (M+K)

### Example 10

### Preparation of (2R,3S,4S,5S)-4,5-dihydroxy-1-[4'-(4"-hydroxy- but-2'-ynyloxy)benzenesulfonyl]-3-methoxy-piperidine-2-carboxylic acid hydroxamide:

### (1)(3aS,6R,7S,7aR)-5-(4'-benzyloxybenzenesulfonyl)-2,2-dimethyl-7-methoxy-hexahydro-[1,3]dioxolo[4,5-c]pyridine-6-carboxylic acid methyl ester:

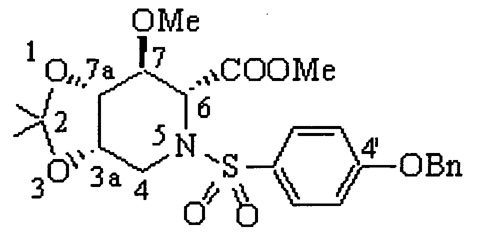

The compound (1.03 g) of Example 8 (1) was dissolved in methylene chloride (10 mL) and methyl iodide (1.35 mL) and silver oxide (1.5 g) were added, followed by stirring under nitrogen atmosphere at room temperature for 5 days. The insoluble material was removed by filtration and the filtrate was concentrated under reduced pressure. The resulting residue was purified by silica gel medium pressure column chromatography (ethyl acetate:cyclohexane = 1:4 → 1:3) to obtain the titled compound (765 mg) as a syrup.
¹H-NMR (CDCl₃)δ: 1.29 (s, 3H), 1.37 (s, 3H), 3.24 (dd, 1H, J = 7.9, 13.2 Hz), 3.46 (s, 3H), 3.61 (dd, 1H, J = 6.6, 13.4 Hz), 3.66 (s, 3H), 4.1-4.15 (m, 2H), 4.27 (q, 1H, J = 6.9 Hz), 4.78 (bs, 1H), 5.12 (s, 2H), 7.04 (d, 2H, J = 8.5 Hz), 7.3-7.45 (m, 5H), 7.83 (d, 2H, J = 8.5 Hz).

### (2) (3aS,4R,7S,7aS)-5-[4'-[4"-(tert-butyldimethylsilanyloxy)-but-2'-ynyloxy]benzenesulfonyl]-7-methoxy-2,2-dimethyl-hexahydro-[1,3]dioxolo[4,5-c]pyridine-6-carboxylic acid methyl ester:

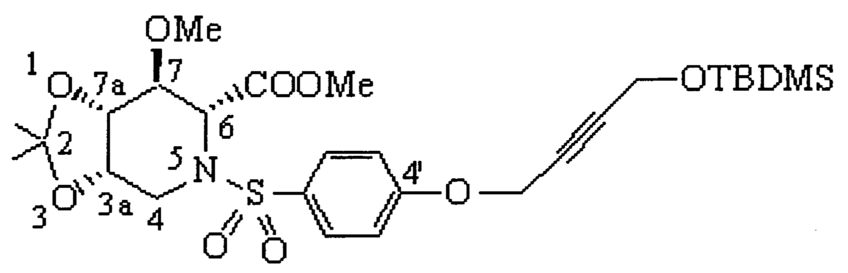

The compound (870 mg) of (1) was dissolved in ethyl acetate (25 mL) and 10% Pd/C (110 mg) was added, followed by stirring under hydrogen atmosphere at room temperature for 3.5 hours. The catalyst was removed by filtration and the filtrate was concentrated under reduced pressure. The resulting residue was dissolved in methylene chloride (8 mL) and (4-bromo-but-2-ynyloxy)-tert-butyldimethylsilane (700 mg) and silver oxide (616 mg) were added, followed by stirring under nitrogen atmosphere at 50°C overnight. The insoluble material was removed by filtration and the filtrate was concentrated under reduced pressure. The resulting residue was purified by silica gel medium pressure column chromatography (ethyl acetate:cyclohexane = 1:3) to obtain the titled compound (490 mg) as a syrup.
¹H-NMR (CDCl₃) δ: 0.093 (s, 6H), 0.89 (s, 9H), 1.29 (s, 3H), 1.37 (s, 3H), 3.24 (dd, 1H, J = 7.9, 13.2 Hz), 3.47 (s, 3H), 3.60 (dd, 1H, J = 6.5, 13.2Hz), 4.1-4.2 (m, 1H), 4.27 (q, 1H, J = 6.8 Hz), 4.35 (s, 2H), 4.78 (bs, 3H), 7.03 (d, 2H, J = 8.5 Hz), 7.84 (d, 2H, J = 8.5 Hz).

### (3) (3aS,4R,7S,7aS)-5-[4'-[4"-(tert-butyldimethylsilanyloxy)-but-2'-ynyloxy]benzenesulfonyl]-7-methoxy-2,2-dimethylhexahydro-[1,3]dioxolo[4,5-c]pyridine-6-carboxylic acid hydroxamide:

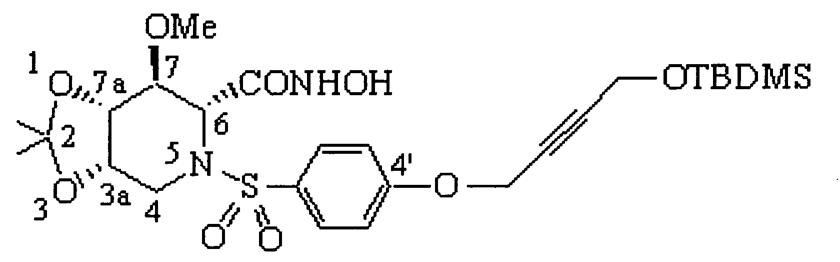

The compound (487 mg) of (2) was dissolved in methanol (20 mL) and an aqueous 50% hydroxylamine solution (4 mL) and sodium cyanide (41 mg) were added, followed by stirring at room temperature for 2 days. The reaction solution was distilled off under reduced pressure and the resulting residue was purified by silica gel medium pressure column chromatography (chloroform:methanol = 50:1 → 35:1) and then freeze-dried to obtain the titled compound (122 mg) as an amorphous.
¹H-NMR (DMSO-d₆) δ: 0.04 (s, 6H), 0.84 (s, 9H), 1.18 (s, 3H), 1.36 (s, 3H), 3.25 (s, 3H), 3.2-3.3 (m, 1H), 3.55-3.7 (m, 3H), 3.76 (d, 1H, J = 7.2 Hz), 4.09 (q, 1H, J = 7.5 Hz), 4.35 (s, 2H), 4.99 (s, 2H), 7.16 (d, 2H, J = 8.7 Hz), 7.82 (d, 2H, J = 8.7 Hz), 9.05 (s, 1H), 10.62 (s, 1H).
TOF-Mass: 607 (M+Na), 623 (M+K)

### (4) (3aS,4R,7S,7aS)-5-[4'-(4"-hydroxy-but-2'-ynyloxy)benzenesulfonyl]-7-methoxy-2,2-dimethyl-hexahydro-[1,3]dioxolo[4,5-c]pyridine-6-carboxylic acid hydroxamide:

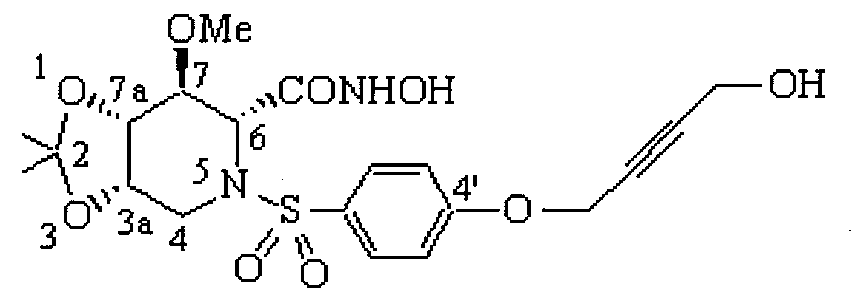

The compound (121 mg) of (3) was dissolved in THF (5 mL) and acetic acid (34 mg) was added, followed by the addition of TBAF (0.57 mL) and further stirring at room temperature for 3 hours. The reaction solution was distilled off under reduced pressure and the resulting residue was purified by silica gel medium pressure column chromatography (chloroform:methanol = 50:1 → 25:1 → 15:1) and then freeze-dried to obtain the titled compound (70 mg) as an amorphous.
¹H-NMR (DMSO-d₆) δ: 1.19 (s, 3H), 1.36 (s, 3H), 3.25 (s, 3H), 3.2-3.3 (m, 1H), 3.62 (t, 1H, J = 7.1 Hz), 3.65-3.7 (m, 2H), 3.76 (d, 1H, J = 7.2 Hz), 4.05-4.15 (m, 3H), 4.97 (s, 2H), 5.26 (t, 1H, J = 5.7 Hz), 7.16 (d, 2H, J = 8.9 Hz), 7.82 (d, 2H, J = 8.9 Hz), 9.05 (s, 1H), 10.61 (s, 1H).
TOF-Mass: 493 (M+Na), 509 (M+K)

### (5) (2R,3S,4S,5S)-4,5-dihydroxy-1-[4'-(4"hydroxy-but-2'-ynyloxy)benzenesulfonyl]-3-methoxy-piperidine-2-carboxylic acid hydroxamide:

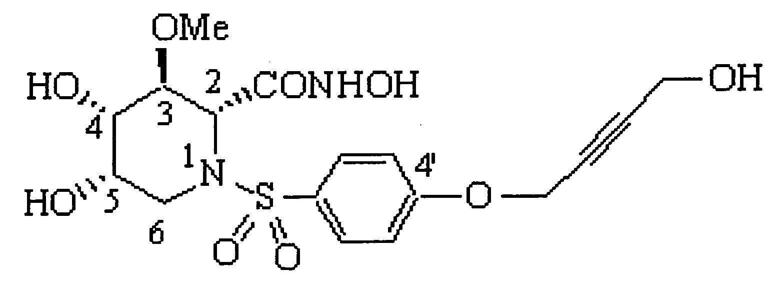

The compound (65 mg) of (4) was dissolved in methanol (6 mL) and a cation exchange resin (MUROMAC, 1.5 g) was added, followed by stirring at room temperature overnight. The insoluble material was removed by filtration and the filtrate was concentrated under reduced pressure. The resulting residue was purified by silica gel medium pressure column chromatography (chloroform:methanol = 20:1 → 6:1 → 5:1) and then freeze-dried to obtain the titled compound (17 mg) as an amorphous.
¹H-NMR (DMSO-d₆) δ: 3.21 (s, 3H), 3.2-3.3 (m, 1H), 3.43 (bs, 1H), 3.61 (bs, 1H), 4.11 (d, 1H, J = 5.9 Hz), 4.27 (s, 1H), 4.75 (d, 1H, J = 4.8 Hz), 4.94 (s, 2H), 5.27 (t, 1H, J = 6.0 Hz), 7.10 (d, 2H, J = 8.7 Hz), 7.73 (d, 2H, J = 8.7 Hz), 8.86 (s, 1H), 10.63 (bs, 1H).
TOF-Mass: 453 (M+Na), 469 (M+K)

### Example 11

### Preparation of tablets

In the following manner, tablets, each of which contains 100 mg of (2R,3S,4R,5S)-1-(4'-but-2'-ynyloxybenzenesulfonyl)-3,4-dihydroxy-5-methoxypiperidine-2-carboxylic acid hydroxamide (compound c) of Example 4, are obtained.

### [Formulation]

| Ingredients | Amount |
|---|---|
| Basis (compound c) | 100 Parts by weight |
| Cornstarch | 46 Parts by weight |
| Microcrystalline cellulose | 98 Parts by weight |
| Hydroxypropylcellulose | 2 Parts by weight |
| Magnesium stearate | 4 Parts by weight |

### [Operation]

A basis, cornstarch and microcrystalline cellulose are mixed and a solution prepared by dissolving hydroxypropylcellulose in 50 parts by weight of water is added, followed by well kneading. The resulting kneaded mixture is passed through a sieve, granulated and then dried to obtain granules. The resulting granules are mixed with magnesium stearate and then the mixture is compressed into tablets each having a weight of 250 mg.

### Example 12

### Preparation of granules

In the following manner, granules containing (2R,3S,4R,5S)-1-(4'-but-2'-ynyloxybenzenesulfonyl)-3,4-dihydroxy-5-methoxypiperidine-2-carboxylic acid hydroxamide (compound c) of Example 4 are obtained.

### [Formulation]

| Ingredients | Amount |
|---|---|
| Basis (compound c) | 200 Parts by weight |
| Lactose | 185 Parts by weight |
| Cornstarch | 109 Parts by weight |
| Hydroxypropylcellulose | 6 Parts by weight |

### [Operation]

A basis, lactose and cornstarch are mixed and a solution prepared by dissolving hydroxypropylcellulose in 120 parts by weight of water is added, followed by well kneading. The resulting kneaded mixture is passed through a #20 mesh sieve, granulated, dried and then sized to obtain granules containing 200 mg of a basis in 500 mg.

### Example 13

### Preparation of capsules

In the following manner, capsules, each of which contains 100 mg of (2R,3S,4R,5S)-1-(4'-but-2'-ynyloxybenzenesulfonyl)-3,4-dihydroxy-5-methoxypiperidine-2-carboxylic acid hydroxamide (compound c) of Example 4, are obtained.

### [Formulation]

| Ingredients | Amount |
|---|---|
| Basis (compound c) | 100 Parts by weight |
| Lactose | 35 Parts by weight |
| Cornstarch | 60 Parts by weight |
| Magnesium stearate | 5 Parts by weight |

### [Operation]

The above respective ingredients are well mixed and each 200 mg of the resulting powder mixture is filled in a capsule to obtain a capsule.

### Example 14

### Preparation of injections

A mixture of 0.5 parts by weight of (2R,3S,4R,5S)-1-(4'-but-2'-ynyloxybenzenesulfonyl)-3,4-dihydroxy-5-methoxypiperidine-2-carboxylic acid hydroxamide (compound c) of Example 4 and 5 parts by weight of sorbitol is dissolved in distilled water for injection to make 100 parts by weight and then the aqueous solution is filtered through a membrane filter. Each 5 g of the filtrate is filled in an ampoule subjected to a treatment of replacing by nitrogen. After melt sealing, the ampoule is sterilized at 120°C for 15 minutes to obtain an injection containing 25 mg of a compound c in an ampoule.

### Example 15

### Preparation of ointments

1.0 Parts by weight of (2R,3S,4R,5S)-1-(4'-but-2'-ynyloxybenzenesulfonyl)-3,4-dihydroxy-5-methoxypiperidine-2-carboxylic acid hydroxamide (compound c) of Example 4 and 0.1 parts by weight of butylparaben are dispersed in 5.0 parts by weight of light liquid paraffin, ground in a mortar and then passed through a #200 mesh sieve. The resulting product is mixed with 5.0 parts by weight of liquid paraffin and then uniformly dispersed in 88.9 parts by weight of gelled hydrocarbon heated to about 60°C to obtain oily ointments.

### INDUSTRIAL APPLICABILITY

The compound of the present invention exerts a potent inhibitory effect on TACE, while it hardly exerts a inhibitory effect on MMP1, MMP3 and MMP9 (see Test Examples). Therefore, the compound of the present invention is extremely useful as a TACE inhibitor.

## Claims

1. A compound represented by the general formula (I): 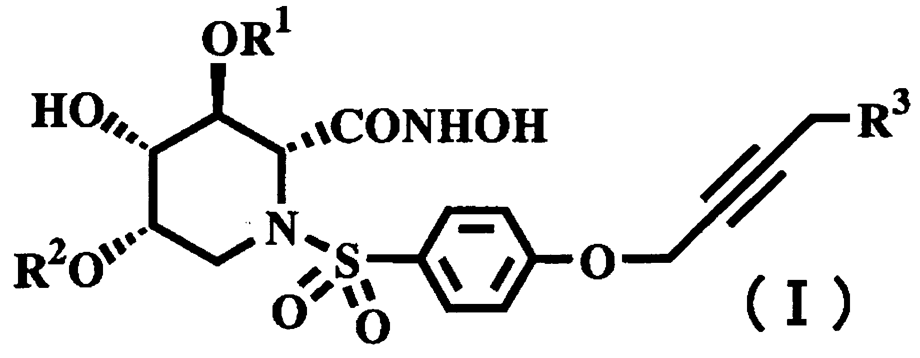 wherein R¹ and R² represent a hydrogen atom, a C₁-C₈ alkyl group, a C₃-C₈ alkenyl group or a benzyl group and at least one of R¹ and R² is a hydrogen atom, and R³ represents a hydrogen atom or a hydroxyl group, or a pharmaceutically acceptable salt thereof.

2. A drug comprising the compound (I) according to claim 1, or a pharmaceutically acceptable salt thereof, as an active ingredient.

3. A TACE inhibitor comprising the compound (I) according to claim 1, or a pharmaceutically acceptable salt thereof, as an active ingredient.

4. A compound represented by the following formula (IIA) or (IIB) : 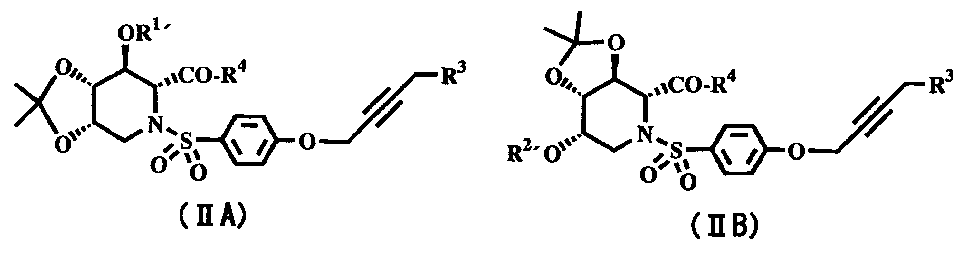 wherein R¹' and R²' represent a hydrogen atom, a C₁-C₈ alkyl group, a C₃-C₈ alkenyl group or a benzyl group, R³ represents a hydrogen atom or a hydroxyl group, and CO-R⁴ represents a hydroxamic acid equivalent.
